# EUROPEAN PATENT SPECIFICATION

(11) **EP 2 837 359 B1**
(45) Date of publication and mention of the grant of the patent: **10.06.2020**
(21) Application number: 13775778.7
(22) Date of filing: 11.04.2013
(51) Int. Cl.: A61F 2/86, A61L 31/14, C23C 8/26, A61L 27/04, A61L 27/58, A61L 31/02

(54) **BIOABSORBABLE MEDICAL DEVICE OR MEDICAL DEVICE COMPONENT AND PREPARATION METHOD THEREOF**
BIOABSORBIERBARE MEDIZINISCHE VORRICHTUNG ODER KOMPONENTE EINER MEDIZINISCHEN VORRICHTUNG UND HERSTELLUNGSVERFAHREN DAFÜR
DISPOSITIF MÉDICAL BIOABSORBABLE OU COMPOSANT DE DISPOSITIF MÉDICAL ET PROCÉDÉ DE PRÉPARATION ASSOCIÉ

(30) Priority: 12.04.2012 CN 201210106761
(43) Date of publication of application: 18.02.2015
(73) Proprietor: Lifetech Scientific (Shenzhen) Co., Ltd., 518057 Nanshan District, Shenzhen (CN)
(72) Inventor: WANG, Wenbin, 518057 Nanshan District, Shenzhen (CN); ZHANG, Deyuan, 518057 Nanshan District, Shenzhen (CN); LIU, Xiangdong, 518057 Nanshan District, Shenzhen (CN); LIN, Wenjiao, 518057 Nanshan District, Shenzhen (CN)
(74) Representative: Prinz & Partner mbB
(86) International application number: PCT/CN2013/074065
(87) International publication number: WO 2013/152728

(56) References cited:
- WO-A1-2011/155063
- CN-A- 101 374 479
- CN-A- 101 549 170
- CN-A- 101 549 170
- CN-A- 101 808 595
- CN-U- 202 821 735
- US-A1- 2010 087 910
- US-A1- 2011 077 732
- Anonymous: "Vickers hardness test - Wikipedia", , 3 February 2017 (2017-02-03), XP055375504, Retrieved from the Internet: URL:https://en.wikipedia.org/wiki/Vickers_ hardness_test#cite_note-5 [retrieved on 2017-05-23]

## Description

The present invention relates to a bioabsorbable stent and a preparation method thereof, in particular to a multi-layer structure of a nitrided bioabsorbable vascular stent and a preparation method thereof.

### Background art

In 1977, Gruentzig underwent the first case of percutaneous transluminal coronary angioplasty (PTCA), breaking through the therapy situation of drugs and surgery and creating a new era of interventional cardiology. Since the development of interventional therapy of coronary heart diseases, a percutaneous transluminal coronary angioplasty (PTCA) era, a bare metal stent (BMS) era and a drug-eluting stent (DES) era have been experienced. The vascular restenosis rate is reduced to below 10 percent by a drug stent from 50 percent of balloon dilatation alone and 20 to 30 percent of a bare metal stent. Especially, when small vascular diseases or long lesions are suffered, the advantages of the drug stent are very obvious.

The coronary stents commonly used in clinic falls into two categories: bare metal stents and drug-eluting stents. Currently, the market share of the drug-eluting stents reaches 95% in China, but in foreign countries, the market share of the bare metal stents is still 30% to 50%. This is because that although the restenosis and revascularization rates can be reduced by the DES, the existing drug stents with polymer carrier still have some limitations, mainly showing late and very late stent thrombosis problems, delayed endothelial healing and late catch-up of lumen loss, and the main reason is polymer carrier-induced inflammation. The problems and the effective means for solving the problems have been widely debated in the field of international research. One research field is to develop a fully biodegradable polymer coating drug-loaded stent, and the other development field is to avoid the use of a polymer coating, i.e., a carrier free drug stent. However, since a substrate material becomes a permanent implant, its long-term potential risks still exist.

The therapy method of infant congenital vascular stenosis (coarctation of the aorta and pulmonary stenosis) includes surgery, balloon angioplasty and stent implantation. Although surgery is a good method, it is a thoracotomy and has a big trauma; meanwhile it is difficult to solve the pulmonary branch stenosis and postoperative restenosis. The balloon angioplasty and the stent implantation, which are safe transcatheter interventional therapy methods, have advantages of less trauma and shorter hospital stay, etc.; but the balloon angioplasty leads to a higher incidence of complications, especially for babies; therefore, the stent implantation is proved to be a better choice. However, infants have further growth and development characteristics, and the non-absorbable stents implanted can cause restenosis in the late period of vascular growth; although the stent diameter can be consistent with the vascular growth by a re-expansion method, adult stents can not be implanted into blood vessels of infants because blood vessels of infants are thinner.

Currently, the bioabsorbable vascular stent has become a research focus, and has the advantages that the ordinary stents do not have: further growth of blood vessels and the follow-up vascular surgical therapy can not be hindered; after the bioabsorbable stent is completely absorbed by the human body, narrow blood vessels will be restored to healthy and natural normal blood vessels with a physiological vasomotor capacity; the stent can be fully absorbed until the stent disappears completely, so that the chronic injury and inflammatory reaction caused by the stent for a long term can be avoided and the late stent thrombosis is reduced and so anti-platelet drugs do not need to be taken for a long term; once the stent is completely absorbed, the stent does not have the long-term potential adverse effects on the blood vessels without increasing the surgery difficulty of re-PCI or surgical revascularization, which has a great significance, especially for the blood vessels of children in the period of growth and development.

The bioabsorbable stents mainly comprise a polymer-based bioabsorbable stent and a metal-based bioabsorbable stent. But the former has unsatisfactory biomechanical properties, and simultaneously the complexity of such stent release process is much higher than that of the conventional balloon dilatation metal stent. The latter mainly includes a magnesium alloy stent and an iron stent at present. The magnesium alloy stent can not play an effective supporting role before revascularization due to its too fast corrosion rate; therefore, the development focus of the magnesium alloy stent lies in how to reduce the corrosion rate thereof. Pure iron applied to the bioabsorbable stents has the main disadvantages of low mechanical properties and too slow corrosion rate. In the prior art, a composite coating containing strontium or calcium, or both is prepared on the surface of bioabsorbable metal materials such as pure iron by physical vapor deposition to accelerate and control the corrosion rate of the materials. In addition, a polymer coating which can be degraded in an acidic environment is sputtered on the composite coating to further accelerate the corrosion rate of the materials. However, such a method fails to solve the problems of low mechanical properties of pure iron materials. The coating and substrate pure iron have a problem on whether both are firmly bound or not due to their non-integrated structure.

The current research mainly focuses on developing novel iron-based alloys and finding novel iron material preparation methods, or preparing an iron alloy layer on the surface of the pure iron material and modifying the pure iron material in order to solve the problems of the pure iron stents; wherein the pure iron stent is subjected to surface alloying treatment (carburizing, nitriding, and carbonitriding) to obtain a composite diffusion layer with an adjustable permeation depth, thereby improving the strength of the stent, and simultaneously accelerating the corrosion rate of the stent and shortening the absorption cycle of the stent. After the stent is subjected to surface alloying, the stent has a non-continuous diffused composite diffusion layer. By controlling the distribution, shape and depth of the diffusion layer, the yield strength and elongation can be adjusted in a wide range to achieve the strength and absorption cycle required by the stent. The composite diffusion layer comprises a solid solution that nitrogen exists in iron, and Fe₄N.

The bioabsorbable metal represented by pure iron and magnesium alloy can be used for manufacturing other implantable medical devices other than for manufacturing bioabsorbable vascular stents.

The vascular stent subjecting to a surface treatment method such as nitriding, carburizing or carbonitriding and then being polished in the prior art has a composite diffusion layer with the adjustable permeation depth. An unsolved technical problem is how to optimize the structure on this basis to improve the comprehensive properties (radial strength, flexibility, fatigue resistance and corrosion rate) of the absorbable stent.

The radial strength of the stent is defined herein as a pressure required when the stent radially deforms by 10%. For a coronary stent with an outer diameter of 1.6mm, the outer diameter is dilated to 3.0mm by a balloon generally and then the radial strength is measured. A 316L stainless steel coronary stent has a wall thickness of about 100µm usually, and a radial strength ranging from 110KPa to150KPa; the mechanical properties of cobalt-chromium alloy is slightly better than those of 316L stainless steel, and a cobalt-chromium alloy coronary stent has a wall thickness of about 80µm usually with a radial strength ranging from 140 KPa to 185KPa.

The blood vessels of human body usually tend to be bent or twisted, especially a vascular lesion segment. Flexibility of the stent refers to a capacity of the stent to adapt to bent blood vessels. The better the stent flexibility is, the stronger the penetration capacity of the stent through the blood vessels is. According to the finite element analysis of the stent, a wall thickness of the stent not only is a main factor affecting the stent flexibility, but also one of key parameters reflecting the comprehensive properties of the stent. Meanwhile, the wall thickness of the stent is regarded as an independent predictive factor of late lumen loss (vascular restenosis) after vascular lesions are treated by an interventional therapy, and the evidence-based medicine agrees that the restenosis rate of a thin-walled stent is lower than that of a thick-walled stent. However, the wall thickness of the stent is reduced at the expense of the loss of radial strength of the stent; therefore the wall thickness of the stent is strictly limited by clinical requirements. The commonly used coronary stents are all permanently implantable, including bare metal stents and drug-eluting stents, in which 316L stainless steel or cobalt-based alloy are usually taken as a substrate material. However, the mechanical properties of the stent depend on the substrate material and the stent structure design, i.e., the mechanical properties of the coronary stent can not be affected by drugs basically. Under the premise of ensuring the clinical therapeutic effects, the wall thickness of the current permanently implantable coronary stent can only be reduced to 65µm, and the stent uses cobalt-chromium alloy as the substrate material. Under the same mechanical property requirements, although the wall thickness of the iron-based coronary stent is significantly less than that of the coronary stents made from other bioabsorbable materials, the wall thickness of the bioabsorbable iron-based coronary stent obtained by the prior art can only be reduced to 90µm or so, and does not reach the minimum wall thickness of 65µm of the permanently implantable coronary stent. The technical problem on how to reduce the wall thickness of the bioabsorbable iron-based stent as much as possible under the premise of satisfying the mechanical properties such as elongation and radial strength and improving the corrosion rate of the stent has not been solved yet by the prior art.

The iron-based material (including pure iron, steel or other iron alloys) is subjected to a surface nitriding process such as ion nitriding, so that a denser compound layer is formed on the surface of the iron-based material generally. According to the known research results, the compound layer is formed by γ' phase (mainly Fe₄N), or by mixing γ' phase and ε phase (composition change range Fe₂₋₃N); wherein the γ' phase accounts for 50 to 100% by weight. The nitrogen content of the γ' phase is 6 wt-% or so, and the nitrogen content of the ε phase is 8 to 11 wt-% or so; therefore, nitrogen atoms in the compound layer have a very high concentration, and are diffused to inside of the material through high temperatures. The compound layer is easily formed on the surface of the iron-based stent after the iron-based stent is subjected to surface nitriding treatment, and can greatly increase brittleness of the material; the corrosion resistance of the compound layer is much higher than that of a pure iron substrate. Therefore it is necessary to fully remove the compound layer under the premise of ensuring the properties of the stent. When the iron-based stent is prepared by a prior method, if the plasma discharge bias is too low (below 600V), the average thickness of the compound layer is generally more than 10µm. If the temperature of the iron-based stent is too high (above 550°C), the local compound layer on the surface of the iron-based stent extends to inside of the material in a dendritic or flaky shape, resulting in very uneven thickness of the compound layer. In the prior art, a pure iron pipe subjected to drawing and mechanical polishing is used, and the grain boundary is in a disordered high-energy state due to work hardening; meanwhile, there are higher internal residual stress inside the pipe and more defects such as dislocations, thereby providing more express channels for diffusion of nitrogen atoms; the compound layer will extend to a deep part inside the iron pipe material along the grain boundary or a dense dislocation area to show an inward dendritic morphology. The crystal defects of the surface can be significantly reduced by fully annealing the pure iron pipe, but this is not conducive to the permeation of nitrogen ions; therefore it is difficult to solve the problem. In view of the subsequent imprecise and non-uniform surface polishing treatment (the larger the thickness is removed by polishing, the more unfavorable), a compound layer with a certain thickness or higher coverage rate is possibly remained after the surface of such stent is polished; therefore, the purpose of improving the corrosion rate of the iron-based stent may not be achieved.

After being implanted into the blood vessel, the absorbable stent must maintain sufficient mechanical properties within an initial period of time (several months or longer) to adapt to the bending shape of the blood vessel and block the collapse of lesion blood vessel, and can be gradually absorbed after the vascular remodeling is stable. If the local strain of the stent exceeds a certain limit, micro-cracks will be firstly generated on the surface of a part of supporting rod or connecting part. Due to vascular pulsating and blood flow, the metal fatigue of the stent will be gradually accumulated; at this time, the micro-cracks will gradually propagate to inside from the surface of the stent to become larger cracks damaging the stent structure until the stent is partially fractured, and it is even more necessary to prevent the propagation of the micro-cracks for the thin-walled stent. Therefore, the prior art needs to be optimized to ensure that sufficient pure iron or a low-nitrogen-content area is still reserved inside the nitrided stent substrate to reduce the risks of surface crack propagation and premature brittle failure of the stent (before the vascular remodeling is stable). Hence, the ratio of the depth of a nitrided layer to the wall thickness of the stent can not be too large.

In order to obtain a better biological tissue compatibility, the roughness of inner and outer surfaces of the coronary stent should be reduced as much as possible. The ordinary electrochemical polishing used in the prior art does not have a good polishing effect on the inner wall of the stent, and the surface roughness can only be controlled below 0.1µm, which cannot reach a mirror bright effect (surface roughness ≤0.01µm); the stent surface can be bright and smooth when the polishing removal amount (the difference value between wall thicknesses of a polished stent and an unpolished stent) reaches 40µm above in the prior art; thus the inherent shortcomings of imprecision and unevenness of polishing treatment are more obvious, and especially not conducive to the quality control of thin-walled coronary stents.

An important technical problem related to this is that the prior art is difficult to be used for a thin-walled pipe (wall thickness is less than 100µm). If the original pipe wall of the stent pipe is very thin, the nitrided layer is bound to be thinner. However, in order to achieve the desired polishing effect, the prior polishing method requires a higher polishing removal amount, the compound layer closest to the surface can not only be removed, but also a part of nitrided layer can be removed, so that the remaining nitrided layer will become very thin. The thickness uniformity of the nitrided layer is limited by the prior art, the polishing removal amount of different areas of the supporting rod are not uniform enough, and the two uniformities are superimposed together, resulting in the more non-uniform thickness of the remaining nitrided layer. If the remaining nitrided layer is too thin, the nitrided layers at some parts of the supporting rod will almost completely disappear, thereby bringing serious adverse effects. The nitrided layer can effectively improve the radial strength of the stent, which is especially critical to the thin-walled stent; if the thickness of the nitrided layer is not very uniform, the mechanical properties of the various parts of the stent are very inconsistent; thus, the radial strength of the stent will not meet the design requirements. In addition, if the nitrided layer is very thin and has a non-uniform thickness, some parts of the supporting rod are slowly corroded due to less nitrogen content; thus the stent can also not meet the design requirements. By further considering the process error in actual production, the difference between distant supporting rods or connecting parts will be more obvious, and the problems of inconsistency of the above-mentioned mechanical properties or less local nitrogen content will be more serious. Therefore, the polishing process in the prior art needs to be improved so as to adapt to the thin-walled pipe with a wall thickness of less than 100µm.

US 2011/0077732 A1 discloses a method for heat treating an implant in a nitrogen atmosphere at a temperature of 500 to 530 degrees C to from a nitrogen rich near-surface boundary having a maximum thickness of 150µm. The boundary layer has an inner zone representing a diffusion layer, characterized by the formation of nitride needles. The adjacent outer nonmetallic zone, about 5 to 30µm thick, is nitrogen rich and composed essentially of γ-nitrides and β-nitrides of iron.

CN 101 549 170 A discloses a method of forming a stent in a surface alloying treatment using a nitriding process between 400 and 550 degrees C to form a diffusion layer. The layer has an adjustable thickness and comprises a solid solution of nitrogen and Fe₄N. A typical wall thickness of the stent is about 200µm and a final polishing step is performed electro-chemically for a duration of 3 min.

### Technical problems

The technical problems to be solved by the present invention are to provide a bioabsorbable vascular stent and a preparation method thereof so as to solve the problems on the conflicting characteristics among corrosion rate, radial strength, flexibility and wall thickness of the bioabsorbable medical device or medical device component in the prior art being difficult to achieve a relatively perfect balance point.

According to the present invention, a bioabsorbable vascular stent is provided as defined in claim 1. The stent includes an absorbable component produced by subjecting a prefabricated component made from an iron-based raw material to ion nitriding treatment. The material composition inside the absorbable component changes with the depth from the surface. The absorbable component comprises at least a first part and a second part. The second part is surrounded by the first part. The hardness of the first part is higher than that of the second part. An interface is arranged between the first part and the second part. Cracks generated in the first part are obstructed by the interface when propagating to the second part.

The absorbable component comprises a diffusion layer which is continuously distributed from the surface, but not a corrosion resistant compound layer, the diffusion layer comprises a solid solution with a nitrogen content of less than 1 wt-%, and particles with a nitrogen content of more than 1 wt-%; the particles are diffused in the solid solution; the absorbable component also comprises a solid solution layer surrounded by the diffusion layer; the first part comprises the whole diffusion layer; the second part comprises the whole solid solution layer, and the diffusion layer and the solid solution layer are respectively positioned at the two sides of the interface.

The absorbable component comprises a diffusion layer which is continuously distributed from the surface, but does not have a corrosion resistant compound layer formed of γ-phase and ε-phase nitrides.

As a further embodiment, the nitrogen content of the diffusion layer is between 1 wt-% and 3.7 wt-%.

As a further embodiment, the particles account for not more than 63% by mass ratio of the diffusion layer.

As a further embodiment, the sizes of most particles are between 30nm to 2µm, a part having a maximum Vickers hardness of the absorbable component is positioned nearby the surface of the absorbable component and a maximum hardness does not exceed 350HV0.01 a hardness of each part of the diffusion layer is increased by 50HV0.01 compared with the hardness of the non-nitrided iron-based raw material of the prefabricated component.

According to the present invention, the Vickers hardness of the diffusion layer is more than 220HV0.01 and less than 320HV0.01 and gradually decreases with the depth.

As a further embodiment, the sizes of most particles are between 30nm and 500nm.

As a further embodiment, the thickness of the diffusion layer accounts for 75% to 90% of the thickness of the absorbable component.

As a further embodiment, the absorbable component further comprises a core layer surrounded by the solid solution layer; the hardness of the solid solution layer is increased by 10HV0.01 compared with the hardness of the non-nitrided iron-based raw material of the prefabricated component, the thickness of the diffusion layer accounts for 50% to 70% of the thickness of the bioabsorbable medical device or medical device component, and the thickness of the solid solution layer accounts for 15% to 25% of the thickness of the bioabsorbable medical device or medical device component.

As a further embodiment, the absorbable component comprises a plurality of supporting rods connected to form a tubular grid, the vascular stent is produced by subjecting an iron-based pipe with a uniform wall thickness to engraving and ion nitriding treatment, and the wall thickness of the vascular stent is between 60µm and 300µm; under the conditions of same tubular grid and wall thickness, the radial strength of the vascular stent is increased by 30% above compared with the radial strength of the non-nitrided vascular stent prefabricated by the iron-based raw material.

As a further embodiment, the vascular stent has a wall thickness of between 60µm and 300µm, and a mass of between 5mg and 100mg.

As a further embodiment, the vascular stent has a wall thickness of between 60µm and 100µm or 160µm and 170µm.

As a further embodiment, the vascular stent has a Vickers hardness of between 280HV0.01 and 320HV0.01; under the conditions of same tubular grid and wall thickness, the radial strength of the vascular stent is increased by 80% above compared with the radial strength of the non-nitrided vascular stent prefabricated by the iron-based raw material.

As a further embodiment, the electrochemical corrosion surface current density of the bioabsorbable medical device or medical device component is increased by 20% above compared with the electrochemical corrosion surface current density of the non-nitrided iron-based raw material.

The present invention further provides a preparation method of a bioabsorbable vascular stent as defined in claim 8. The method comprises the steps of:
1.taking pure iron or an iron alloy with an iron containing a wt-% of more than 99% as a raw material to be processed into a prefabricated component, the initial thickness of which is 10µm above greater than a corresponding thickness of the stent;
2. cleaning to remove contaminants on the surface of the prefabricated component;
3. treating the prefabricated component in step 2 by using an ion nitriding method, the prefabricated component having a temperature of between 430°C and 550°C, and nitriding time of between 10 minutes and 200 minutes; wherein during the nitriding step 3, a nitrogen-rich corossion resistant layer (50) is formed on the surface of the component, and
4. subjecting the nitrided prefabricated component to an electrochemical polishing treatment and/or a chemical polishing treatment to completely remove the corrosion resistant layer (50) of the prefabricated component.

As a further embodiment, in step 1, the prefabricated component has a surface Vickers hardness of between 165HV0.01 and 175HV0.01.

As a further embodiment, in step 1, the thickness of the prefabricated component is 20 to 60µm greater than the corresponding thickness of the medical device or medical device component.

As a further embodiment, in step 3, the current density generated on the surface of the prefabricated component by the ion nitriding method is between 0.5mA / cm² and 0.8mA / cm².

As a further embodiment, step 3 further comprises: generating a compound layer on the surface of the prefabricated component, and an average thickness of the compound layer is not more than 5µm.

According to the preparation method of the present invention, step 3 further comprises: generating a diffusion layer and a solid solution layer in the prefabricated component, the solid solution layer is surrounded by the diffusion layer, the hardness of the diffusion layer and the solid solution layer gradually decreases with the depth, and the hardness of each part of the solid solution layer is increased by 10 to 50HV0.01 compared with the hardness of the non-nitrided iron-based raw material of the prefabricated component.

As a further embodiment, step 3 further comprises: further generating a core layer in the prefabricated component, the solid solution layer is surrounded by the diffusion layer, and the core layer is surrounded by the solid solution layer.

As a further embodiment, in step 3, the temperature of the prefabricated component is between 470°C and 520°C.

As a further embodiment, in step 3, the ion nitriding method adopts a mixed gas of nitrogen and hydrogen, the flow ratio of nitrogen to hydrogen is between 1: 2 and 1: 9, and gas discharge is maintained at a pressure of between 40 Pa and 150Pa and a bias of between 600V and 800V.

As a further embodiment, in the step, the flow ratio of nitrogen to hydrogen is between 1: 3 and 1: 7, and gas discharge is maintained at a pressure of between 50 Pa and 100Pa and a bias of between 600V and 650V.

As a further embodiment, in step 4, the electrochemical polishing treatment adopts an electrochemical polishing solution prepared by uniformly mixing anhydrous acetic acid and perchloric acid with a mass concentration of 70% according to a volume ratio of 85:15.

As a further embodiment, in step 4, after the electrochemical polishing treatment, the chemical polishing treatment is carried out again to remove 15 to 25µm outer layer thickness of the prefabricated component.

As a further embodiment, in step 4, the chemical polishing treatment adopts a three-acid system polishing solution having a temperature of between 100°C and 180°C and comprising the following main components of 30% to 55% of H₃PO₄, 20% to 45% of H₂SO₄, 5% to 15% of HNO₃ and 15% to 30% water.

As a further embodiment, in step 4, the time of chemical polishing treatment is between 3 seconds and 8 seconds.

### Beneficial effects of the invention

Compared with the prior art, the bioabsorbable vascular stent provided by the present invention has the following advantages:
1. The absorbable component in the vascular stent is internally provided with a multi-layer structure, and an absorbable stent is taken as an example; the mutually contradictory characteristics of corrosion rate, radial strength, flexibility, and wall thickness of the stent can achieve a relatively perfect balance point; the technical solution of the present invention also provides a preparation method of the absorbable vascular stent, and determines preferred process parameters and corresponding property effects.
2. By using preferable mixed gas flow ratio, air pressure, component temperature, bias, nitriding time and component temperature, the internal structure of the component can be controlled, the thickness of the component layer on the surface of the component is below 5µm, the situation that the compound layer is completely removed is ensured through preferable electrochemical or chemical polishing treatment, both the inner and outer surfaces of the component achieve a mirror bright effect in a low polishing removal amount, and finally, the absorbable components of different internal structures can be obtained.
3. The optimized nitriding process and polishing process provided by the present invention can ensure that under the conditions of same stent grid design and wall thickness, the supporting force of the absorbable iron-based vascular stent (the stent wall thickness can be reduced to 60µm) is comparable with a cobalt-chromium alloy stent. On coronary stents and peripheral stents, such an effect can be obtained; the radial strength of the stent is increased by 31.5% to 94.4% compared with a pure iron stent.
4. The present invention provides the bioabsorbable iron-based vascular stent ; it comprises a multi-layer structure having a gradient property; the supporting force and flexibility of the stent can be simultaneously improved, the corrosion resistance is reduced, the incidence of fatigue cracks in the surface can be reduced, but also the propagation of surface microcracks to the inside of metal can be inhibited, so that the absorbable medical device has a higher fatigue fracture resistant property in the early period when the absorbable medical device is implanted in the human body. Because most of the fatigue cracks are firstly formed in the metal surface, the fatigue crack initiation can be prevented by improving the component surface hardness and refining grains. On the other hand, the improvement on the plasticity of inside of the component will help to inhibit crack propagation; therefore, the propagation of cracks to inside of the component can be effectively inhibited by reserving the solid solution layer or core layer with a lower hardness inside the component. After being subjected to surface nitriding, the pure iron component has a high strength and a high hardness because of the strengthening effect of second-phase particles in the diffusion layer, so that the supporting property of the stent can be effectively improved; the solid solution layer and the core layer keep a higher elongation and a good plasticity similar to pure iron.
5. The corrosion rate of the bioabsorbable vascular stent according to the present invention can be increased by two times or so compared with a pure iron medical device, and the corrosion rate of the bioabsorbable vascular stent in a simulated body fluid PBS can be increased by 180% compared with pure iron; when the outer surface hardness of the diffusion layer is controlled between 230 HV0.01 and 270HV0.01, the finer the second-phase particles in the diffusion layer are, the faster the corrosion rate is. Due to the presence of a certain number of second-phase particles in the diffusion layer, the material is easily subjected to galvanic corrosion when being subjected to normal corrosion, thereby shortening the time required by human body to absorb the medical device.

### Brief Description of the Drawings

Hereinafter, the present invention will be further illustrated with embodiments in conjunction with the accompanying drawings, in which:
Fig.1 is a schematic sectional view of a surface of a nitrided iron material;
Fig. 2 is a schematic sectional view obtained after the surface shown in Fig. 1 is polished for a period of time;
Fig. 3 is a schematic sectional view obtained after the surface shown in Fig. 2 is further polished;
Fig. 4 is a schematic sectional view obtained when the nitrided iron material is polished to an extent meeting requirements;
Fig. 5 is a schematic sectional view of a supporting rod of a vascular stent produced in an embodiment 1;
Fig. 6 is an electron microscopic photograph of a surface of a supporting rod when the vascular stent in the embodiments of the present invention is excessively dilated by 20% by a balloon (micro-cracks are limited in the surface);
Fig.7 is a schematic sectional view of a supporting rod of another produced vascular stent;
Fig. 8 is a schematic sectional view of a supporting rod of another vascular stent obtained by long-time nitriding treatment;
Fig. 9 is a schematic view of a slit formed by locally bending the vascular stent;
Fig. 10 is a schematic view of a uniformly corroded slit of the vascular stent shown in Fig. 9;
Fig. 11 is local shape distortion caused by subjecting the slit in Fig. 9 or Fig. 10 to non-uniform corrosion.

### Description of preferred embodiments.

The present invention is further described in detail in conjunction with the following accompanying drawings and embodiments in order to enable the objects, technical solutions and advantages of the present invention to be more clearly understood. It should be understood that the specific embodiments described herein are merely used to explain the present invention by example and are not intended to limit the present invention.

In the following a balloon dilatation stent is described in detail as an example. The stent comprising a plurality of components is produced by subjecting a round tube material to laser engraving (prefabrication) and then further treatment, and generally, a plurality of supporting rods are connected to form a whole stent grid to form a tubular side wall. The cross section of the supporting rod of the prefabricated stent is approximately rectangular, the cross section of the supporting rod of a finished stent product will lose edges and corners (approximating to an oval or a round shape), and the supporting rod of the absorbable stent is an example of an absorbable component. The thickness of the supporting rod refers to the width of the supporting rod along the diameter direction of the stent; the thicknesses of the supporting rods of the same stent, i.e., the wall thickness of the stent, are approximately same. Flaky connecting parts may be arranged at joints of the plurality of supporting rods, and the wall thicknesses of the connecting parts and the wall thicknesses of other parts (such as supporting rods) of the stent are the same.

The present ion nitriding equipment can also be used for other absorbable implantable devices (such as an occluder) or absorbable components on the implantable medical devices, and can also adopt iron alloys (binary alloys or multicomponent iron alloys such as Fe-Mn alloys and Fe-Mg alloys) to optimize the properties of the absorbable medical device. Based on the method of the present embodiment, for the absorbable component thickness and property requirements of a general implantable medical device, the initial thickness of the prefabricated component should be 10µm above greater than the corresponding thickness of a product component, and the nitriding time of the prefabricated component is selected between 10 minutes and 200 minutes. A positive electrode of a bias power supply and a vacuum chamber of the ion nitriding equipment are connected, or a positive electrode that basically does not hinder air flow is arranged nearby a clamping table, and the prefabricated component and a negative electrode of the bias power supply are connected. A general process comprises the following steps of vacuumizing to below 2Pa, slowly introducing a mixed gas of N₂ and H₂, and maintaining a stable air pressure selected between 40 Pa and 150Pa, preferably a lower air pressure range such as 50 to 100Pa. The bias power supply is turned on, so that the stent surface maintains abnormal glow discharge, and the surface of the prefabricated component is bombarded by nitrogen ions generated by gas ionization so that the temperature of the surface of the prefabricated component rises. The flow ratio of H₂ to N₂ is selected in a range of 1: 2 to 1: 9, and can be replaced with NH₃ or a mixed gas of H₂ and NH₃, and it is easy to maintain the glow discharge under substantially same bias and corresponding air pressure in accordance with the prior art. The highest temperature of the stent is limited to be 550°C, and the claming table can be heated by an auxiliary heating device to indirectly heat the whole stent, and can also be preheated before turning on the bias power supply in order that the temperature of the stent rapidly rises to 500 to 550°C. By adjusting the power of the auxiliary heating device, the temperature of the prefabricated component is stabilized, the gas discharge is also stabilized; meanwhile, the preferable range of bias is between 600V and 650V (up to 800V), and the surface current density of the prefabricated component is preferably between 0.5mA / cm² and 0.8mA / cm² at this time.

### Embodiment 1

According to a well-known stent grid design, the surface of a pure iron pipe was engraved by laser to be prefabricated into a pure iron vascular stent with an outer diameter of 3.6mm, an original wall thickness of 220µm, and a length of 18mm. When the stent was dilated by a balloon to 12mm, the coverage rate of a metal grid on the surface of the stent on the side wall of the stent was 9.6% or so. Then, the stent was treated by the following steps that:

Firstly, the prefabricated pure iron stent was cleaned as follows: the pure iron stent was immersed in an electrochemical polishing solution to be subjected electrochemical cleaning for about 30 seconds at room temperature under the conditions of introducing current of 1.6A and voltage of 12 to 14V or so. The formula of the electrochemical polishing solution was as follows: anhydrous acetic acid and perchloric acid with a mass concentration of about 70% were uniformly mixed according to a volume ratio of 85 to 15. When the stent grid designs of other patterns are used, the metal coverage rate of the surface of the stent may be changed, and meanwhile, the current value only needs to be adjusted according to the change in the metal coverage rate. Through the cleaning step, the wall thickness of the pure iron stent was reduced by 5µm or so (the inner and outer walls were respectively thinned by 2.5µm) in order to completely remove contaminants (including oxides) on the surface of the pure iron stent so as to ensure the effect of subsequent treatment steps. The experiment proves that the surface of the stent is thinned by 1.5 to 3µm to ensure that a fresh clean surface can be obtained so as to fully expose the outer surface of the pure iron substrate material; therefore, the embodiment of the cleaning step can also be replaced by an equivalent means in the prior art, which can be easily implemented by those skilled in the art.

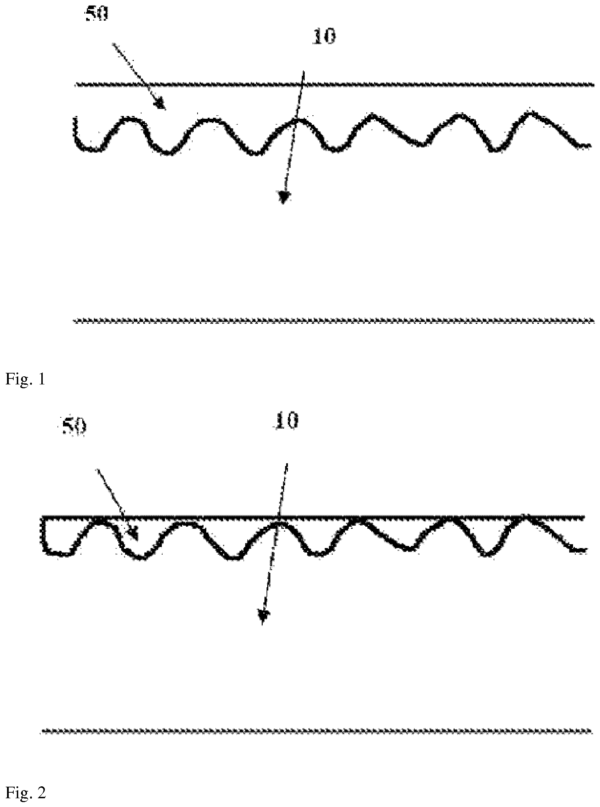

The cleaned pure iron stent was sent to be subjected to surface nitriding treatment, and the stent dehydrated with absolute ethyl alcohol after being cleaned was placed on the insulating clamping table of ion nitriding equipment. During nitriding, a dense nitrogen-rich compound layer 50 with a certain thickness was formed on the surface of the stent, nitrogen atoms in the compound layer 50 at high temperatures were diffused to inside of the stent to form a diffusion layer 10, and the corrosion resistance of the compound layer 50 was much stronger than that of the diffusion layer 10 with less nitrogen content. A local cross-sectional view of the stent was shown in Fig. 1. Because the stent was made from a polycrystalline metal material, in which irregular grain boundaries were distributed, the nitrogen atoms (ions) nearby the outer surface of the compound layer 50 more easily permeate through the diffusion layer 10 from the vicinity of the grain boundaries to generate a nitride, resulting in an interface between the compound layer 50 and the diffusion layer 10 being pushed inwards. The interface undulated in a hill shape. The concentration of nitrogen atoms in the compound layer 50 was high, but the concentration of nitrogen atoms in the diffusion layer 10 was much lower; therefore, the nitrogen atoms were bound to permeate from the compound layer 50 to inside of the diffusion layer.The distribution of the nitrogen atoms depends on concentration gradient, diffusion coefficient, temperature and time. The following parameters were preferably selected in the embodiment: the stent temperature was between 500°C and 520°C, the bias was 600V or so, the flow ratio of nitrogen to hydrogen was 1: 3, the air pressure was 60Pa, the nitriding treatment time was 30 minutes, and the average thickness of the compound layer 50 formed on the surface of the stent was 2µm or so. The concentration of reactive nitrogen ions (nitrogen potential) is appropriately reduced, for example, the flow ratio of nitrogen to hydrogen is adjusted and other parameters are unchanged, so that the generation of the compound layer 50 and the thickness thereof can be effectively controlled. Moreover, the nitrogen ions obtain higher kinetic energy at a higher bias, and meanwhile, the surface sputtering effect of the nitrogen ions on the compound layer 50 is more obvious, so that the outer surface of the compound layer 50 is continuously eroded. Therefore, a lower flow ratio of hydrogen to nitrogen (1: 3 to 1: 5) and a higher bias (600 to 650V) help to inhibit the generation or thickening of the compound layer 50. When the bias is increased, the kinetic energy of nitrogen ions reaching the surface of the stent is increased, the ion bombardment heating effect is enhanced, and the power of the auxiliary heating device is correspondingly reduced in general in order to keep the temperature of the stent unchanged. Under the premise of maintaining the temperature of the stent between 500°C and 550°C, the power of an auxiliary heating power supply is reduced, and the bias is appropriately increased to 700V to 800V (an electric arc is generated due to the surface current of the stent being too large to damage the stent), so that the thickness of the resulting compound layer 50 will be thinner and more uniform.

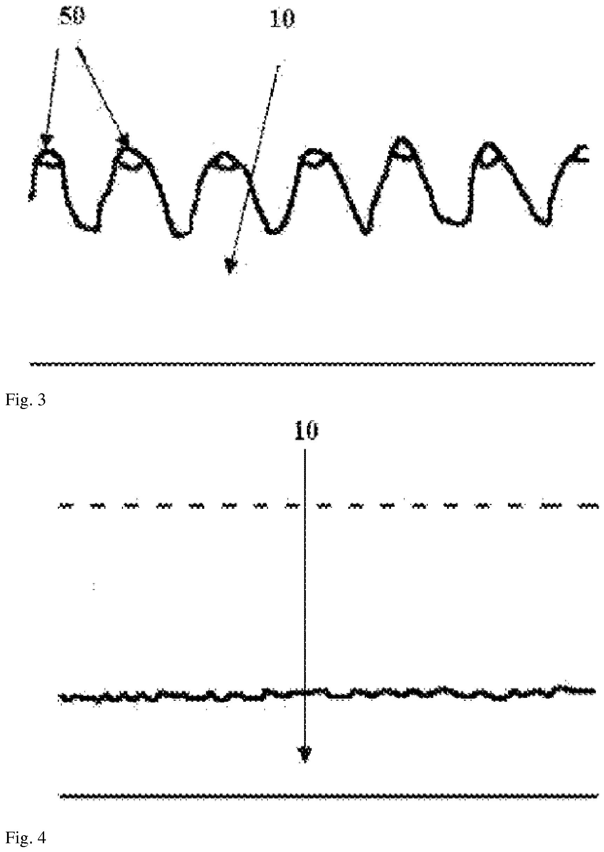

After subjecting to nitriding treatment, the stent was then subjected to polishing treatment in order to completely remove the compound layer 50 and ensure that the surface of the stent was smooth and clean. The stent was placed in the electrochemical polishing solution of the aforementioned formula at a temperature of between 20°C and 25°C, a current of 2.8A, and a voltage of 20 to 23V or so. In this process, it is best to change the electrode clamping direction of the stent so as to ensure that the wall thickness of each part of the stent is uniformly removed. The electrochemical polishing process was divided into three stages. At the first stage, a part of thickness of the compound layer 50 was uniformly removed until the thinnest part of the compound layer 50 was rightly and completely corroded, and a protruding part of the interface of the compound layer 50 still coexisted with the diffusion layer 10, as shown in Fig. 2. At the second stage, the polishing treatment continued to be carried out, the remaining compound layer 50 was gradually thinned to form openings and further became a discontinuous island shape to be continuously shortened; meanwhile, the diffusion layer was corroded more quickly compared with the compound layer 50 due to its poor corrosion resistance, and the exposed outer surface of the diffusion layer 10 was preferentially corroded. As a result, compared with the outer surface of the remaining compound layer 50, the outer surface of the diffusion layer 10 was locally inwards sunken to result in the outer surface of the stent becoming very unsmooth, as shown in Fig. 3. At the third stage, after the remaining compound layer 50 was completely removed, there was only the uneven diffusion layer 10 on the surface of the stent; because the protruding part of the diffusion layer 10 preferentially continued to be corroded compared with a sunken part, the outer surface of the stent was gradually restored to be smooth until a required surface roughness (still unsmooth on a micro level) was reached, as shown in Fig. 4. It can be seen that the final removal amount of electrochemical polishing treatment must be much greater than the maximum thickness of the compound layer 50 due to a great difference between corrosion resistances of the compound layer 50 and diffusion layer 10. Experimental results show that the total thickness (removal amount) removed by electrochemical polishing of the surface of the stent is at least 20µm in order that the compound layer 50 with an average thickness of about 5µm is removed, a mirror similar polishing effect is realized, and the surface roughness Ra is less than 0.01µm. If 20µm thickness of the inner wall surface and 20µm thickness of the outer wall surface of the vascular stent are removed, the actually removed thickness is 40µm ; that is to say, the wall thickness of the pure iron pipe should be increased by 40µm above compared with the wall thickness of a finished stent product in order to ensure that the electrochemical polishing achieves the mirror effect.

In the embodiment, the wall thickness of the stent was expected to be 170µm, the wall thickness of the stent subjecting to cleaning treatment before nitriding was reduced to 215µm, and then 45µm thickness (the inner and outer walls were respectively thinned by 22.5µm) should be removed by using electrochemical polishing treatment after nitriding, thus the polishing time was set to 110 to 120 seconds. Obviously, the polishing treatment was not only limited to a direction perpendicular to the pipe wall of the stent, and about 45µm thickness of each component of the stent in the direction in parallel with the pipe wall of the stent was also removed. Because the polishing treatment was simultaneously carried out in opposite directions at both sides, the actually removed thickness was respectively 22.5µm at each side. If the second stage of the above-mentioned electrochemical polishing treatment is replaced by a chemical polishing method, the corrosion rates of the compound layer 50 and the diffusion layer 10 will be significantly increased and the difference between the two corrosion rates will be decreased; about 10µm wall thickness can be removed for only a few seconds, and recesses in the outer surface of the diffusion layer 10 shown in Fig. 3 will become shallow; the formation process of unsmoothness of the surface of the stent in the second stage will be inhibited to the minimum level, and correspondingly, the removal amount required by the diffusion layer 10 in the third stage will be much less; the removal amount of the whole polishing process can be reduced, the polishing time is shortened accordingly, and a thinner pure iron pipe can be selected. For example, in order that the total removal amount of the polishing treatment in the wall thickness direction is reduced to 25µm and the polishing time is shortened by about half to obtain a stent with a wall thickness of 170µm, a pure iron pipe with a wall thickness of 200µm can be used; accordingly, the production efficiency is improved and the production cost is reduced. After the polishing treatment was finished, the stent was taken out, ultrasonically cleaned in purified water and then naturally dried; preferably, the stent can also be ultrasonically cleaned in absolute ethyl alcohol for 10 minutes in order to avoid the surface of the stent from being oxidized by moisture. Preferably, the stent is firstly cleaned by an alkaline solution before being cleaned by the absolute ethyl alcohol, and the polished stent is placed in a sodium hydroxide solution with a mass percentage of 2% to 8% to be cleaned for about 5 minutes. It was observed under a microscope that the surface roughness Ra of the polished stent could reach 0.01µm below.

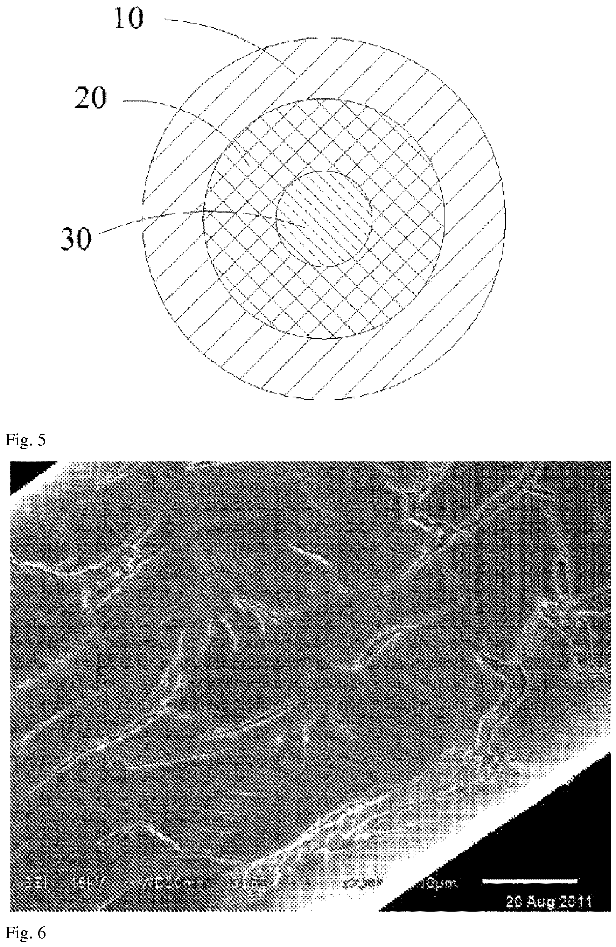

As a preliminary example, the cross section (simplified as a round shape) of a supporting rod of the stent from which the compound layer 50 was completely removed is shown in Fig. 5, because of the specific surface nitriding effect, the diffusion layer 10, the solid solution layer 20 and the core layer 30 were sequentially arranged from outside to inside, the peripheral diameter (i.e., the contour diameter of the cross section of the supporting rod) of the diffusion layer 10 was equivalent to the wall thickness of the stent, the solid solution layer 20 was surrounded by the diffusion layer 10, and the core layer 30 was surrounded by the solid solution layer 20. In fact, the cross-sectional contour of each part of the vascular stent can also be in rectangular, trapezoidal, oval shapes, etc.. The nitriding and polishing were inwardly and simultaneously carried out in a way perpendicular to the outer surface substantially, so that the boundary shapes of the diffusion layer 10, the solid solution layer 20 and the core layer 30 should be similar to the contour of the cross section. It is obviously effective by analogy of the cross sections of other shapes according to the preliminary example and principles and effects thereof. The surface nitriding treatment made the micro-hardness of different regions inside the iron-based medical device different, resulting in obvious difference in technical effects of different regions; that is to say, the relative range of micro-hardness values of the diffusion layer 10, the solid solution layer 20 and the core layer 30 met preset conditions. The micro-hardness was measured by a Vickers hardness test with specific reference to GB/T 11345-2005; however, the stent was too small to bear a usually required test force of 0.3 kg force (2.94N), so the test force was reduced to 0.01 kg force (0.098N). In the embodiment, the raw material of the stent used was a pure iron pipe formed by drawing, the average value of Vickers micro-hardness values was about 170HV0.01 (a micro-hardness value measured by exerting 0.01 kg force on a sample surface and holding for 15 seconds), and the actual hardness value should be between 165HV0.01 and 175HV0.01. The hereinafter called micro-hardness of the pure iron pipe refers to an average value obtained by measuring a plurality of samples, and the micro-hardness of the pure iron pipe is slightly higher than that of the annealed pure iron material (80 to 140HV0 .01) due to cold hardening during drawing molding.

Wherein, the core layer 30 was furthest from the outer surface of the diffusion layer 10, and very few nitrogen atoms could be diffused to the core layer 30 under the set parameter conditions; therefore, the composition of the core layer 30 was similar to the original pure iron pipe, the difference value between the micro-hardness of each part of the core layer 30 and the micro-hardness of the pure iron pipe was less than 10HV0.01, and the hardness of a part close to the center of cross section was lower. The plasticity of the material was quantitatively characterized by elongation and reduction of area in general, and the elongation of the core layer 30 approximated to that of the original pure iron pipe (elongation of 30% to 50%), and therefore, the core layer 30 had a good plasticity similar to the pure iron pipe, and it was very conducive to the prevention of stent surface cracks from propagating to inside of the core layer 30. The main way of metal plastic deformation was slippage on a micro level. The more easily the slippage is generated, the better the plasticity is. The formation of steps on the surfaces of crystals results from slippage, so that the atoms nearby the steps keep binding force; therefore surface cracks cannot be generated. For a stent with a larger wall thickness, such as a peripheral vascular stent, the plasticity of the core layer 30 was particularly important to prevent fatigue fracture of the stent.

At the solid solution layer 20 arranged between the diffusion layer 10 and the core layer 30, there were a certain amount of nitrogen atoms diffused to iron crystals to form approximately saturated solid solutions, or unsaturated solid solutions (not reach saturated solid solubility at room temperature), so that the hardness thereof was significantly improved. The difference value between the micro-hardness of each part of the solid solution layer 20 and the micro-hardness of the pure iron pipe was between 10HV0.01 and 50HV0.01, and the hardness of the solid solution layer 20 also decreased with the depth, i.e., the hardness of a part close to the center of cross section was lower. The elongation of the solid solution layer 20 also approximated to that of the original pure iron pipe, which was conducive to prevent the stent surface cracks from propagating to inside of the solid solution layer 20.

A large number of nitrogen atoms were diffused to the diffusion layer 10 arranged on the periphery of the supporting rod cross section at a high temperature, and the saturated solid solubility of the nitrogen atoms was far more than that of nitrogen atoms in the iron crystals. In the nitriding process until cooling to room temperature, the hardness was significantly increased by the phase change in the diffusion layer 10, and the difference value between the micro-hardness of each part of the diffusion layer 10 and the micro-hardness of the pure iron pipe was more than 50HV0.01. If the micro-hardness of the non-nitrided pipe was 170HV0.01, the micro-hardness of the diffusion layer 10 was preferably in a range of 220 to 280HV0.01, and the highest hardness value occurred nearby the surface. The diffusion layer 10 was formed by subjecting the nitrogen-rich second phase to be diffused to a first phase region in a granular shape; the first phase was saturated solid solutions or approximately saturated solid solutions formed by subjecting the nitrogen atoms to be diffused to the iron crystals, the nitrogen content was less than 1 wt-% (generally less than 0.1 wt-%), and the first phase was continuously filled in the whole region of the diffusion layer 10; the second phase included γ' phase (mainly Fe₄N) and / or α" phase (Fe₁₆N₂), the nitrogen content of the second phase was more than 1 wt-%, the nitrogen contenr of the γ' phase could reach 6 wt-% or so, the nitrogen content of the α" phase could reach 3 wt-% or so, and the second phase accounted for not more than 63 wt-% of the diffusion layer 10. According to the law of diffusion, the concentration of nitrogen atoms in the diffusion layer 10 decreased as the depth (distance) from the surface to inside increased, and the number of the second phase that precipitated from the first phase also decreased as the depth increased. Meanwhile,the diffused second-phase particles could improve the whole hardness of the material. Therefore, the hardness and plasticity of the diffusion layer 10 were gradually changed with the gradient, the hardness was gradually decreased and the plasticity was increased from outside to inside, and the stress was gradually released layer by layer, and thus playing a role in crack propagation resistance to a certain extent. At this time, a part, nearby the surface of the stent, of the diffusion layer 10 had a higher hardness, and a part, farther (deeper) from the surface of the stent, of the diffusion layer 10 had a lower hardness; in fact, the lower-hardness part was surrounded by the higher-hardness part. The cracks generated in the vicinity of the surface of the stent would propagate to the inside; however, certain initial stress was gradually released due to the hardness gradient. The cracks which propagated from the higher-hardness part to the lower-hardness part would stop at a certain position, which could be regarded as the interface between the higher-hardness part and the lower-hardness part; therefore, the further crack propagation was obstructed by the interface. Meanwhile, the outer surface of the diffusion layer 10 had the highest hardness, and thusthe initiation of surface microcracks could be effectively prevented.

In the embodiment, during nitriding, the stent temperature of between 500°C and 520°C or so, the flow ratio of nitrogen to hydrogen of 1: 3, the air pressure of 60Pa and the bias of about 600V were selected, and the polishing treatment was carried out after nitriding for 30 minutes; accordingly, the hardness value of the outer surface of the diffusion layer 10 shown in Fig. 5 was 260HV0.01 or so; wherein the size of second-phase particles was generally 30nm to2µm, the electrochemical corrosion current density of the diffusion layer 10 in the simulated body fluid PBS was about 22µA / cm² and was more than two times higher than that of 10µA / cm² of the pure iron stent (untreated by the method of the embodiment). Preferably, the temperature of the nitrided stent was reduced to 500°C or so, the flow ratio of nitrogen to hydrogen was reduced to 1: 5 to 1: 7, and the air pressure of 60Pa and the bias of about 600V were maintained; accordingly, the diffusion of the nitrogen atoms and the agglomeration and growth of second-phase particles could be slowed down. The nitrogen content of the diffusion layer 10 was in a preferable range of between 1.0 wt-% and 3.7 wt-%, the size of a large number of fine and diffused second-phase particles were basically in a preferable range of between 30nm and 500nm, and the current corrosion density of the diffusion layer 10 in the simulated body fluid PBS was increased to 26µA / cm² or so. If the nitrogen content of the diffusion layer 10 was too low (less than 1.0 wt-%), the number of the formed second phase was too few, micro-galvanic corrosion was only carried out in a small scattered range, and the corrosion rate was not much improved compared with pure iron; if the nitrogen content of the diffusion layer 10 was too high (greater than 3.7 wt-%), and the second phase particles with a size more than 500nm accounted for a significant proportion, leading to the result that the hardness of the outer surface of the diffusion layer 10 was higher than 350HV0. 01 and the galvanic corrosion rate of the outer surface of the diffusion layer 10 was relatively low instead; therefore, the surface hardness of the diffusion layer 10 should be not more than 350HV0.01 and the hardness should gradually decrease towards the inside. Because the second-phase particles might be difficultly corroded, more large-size second-phase particles would eventually hinder the penetration of corrosion. The corrosion rate of the iron-based stent was greatly improved by the preparation method and preferable parameters provided by the embodiment; therefore, the time required by human body to absorb the stent was substantially shortened, which was particularly important to the peripheral vascular stent of larger specification (outer diameter and wall thickness).

Because the micro-hardness of the solid solution layer 20 and the core layer 30 were all far lower than that of the diffusion layer 10, if the proportion of the solid solution layer 20 and the core layer 30 in the total volume of the supporting rod was too large, it was unfavorable to improve the radial strength of the stent. On the other hand, the outer surface of the diffusion layer 10 had a higher micro-hardness, thus helping to prevent micro-crack initiation; however the diffusion layer 10 had a poorer plasticity, thus micro-cracks formed on the outer surface propagated to the inside; if the proportion of the diffusion layer 10 in the total volume of the supporting rod was too large, the fatigue resistance of the stent could be reduced. In order to take into account both the radial strength of the stent and the fatigue resistance thereof, and to better inhibit the surface micro-crack initiation and the inward propagation of micro-cracks, the preferable ratio of the thickness (an average distance between outer surface and inner-side boundary) of the diffusion layer 10 obtained in the embodiment to the radius of the supporting rod was 53% or so, the preferable ratio of the thickness (an average distance between outer-side boundary and inner-side boundary) of the solid solution layer 20 to the radius of the supporting rod was 20% or so, the preferable ratio of the thickness (an average distance between the outer-side boundary and the center) of the core layer 30 to the radius of the supporting rod was 27% or so, and the radius of the supporting rod was half of the wall thickness of the stent. After the stent in the embodiment was dilated by the balloon to 12mm, the radial strength of the stent was measured to be 66KPa which was increased by 46.7% compared with the 45KPa radial strength of a pure iron stent (untreated by the method of the embodiment) of the same shape and size (wall thickness was also 170µm).

The stent temperature during nitriding is a very important parameter. It determines the diffusion rate of nitrogen atoms; and the nitriding time directly affects the thicknesses of the diffusion layer 10 and the solid solution layer 20. In the present embodiment, if the nitriding temperature and the nitriding time were kept unchanged, the flow ratio of nitrogen to hydrogen was reduced to 1: 5 and at the same time the bias was increased to 650 to 700V or the air pressure was increased to 65 to 75Pa, so that the nitriding current density was still maintained basically unchanged, and thus a structure similar to Fig. 5 and a property effect could be still obtained; the flow ratio of nitrogen to hydrogen was further reduced to 1: 5 while the bias was being increased to 750 to 800V or the air pressure was being increased to 120 to 130Pa, the nitriding current density did not change much, thus a structure similar to Fig. 5 and property effect ccould still be obtained. When the air pressure exceeded 150Pa, even if the flow ratio of nitrogen to hydrogen was reduced to 1: 9, the stent surface current density was still too large; the temperature of the stent rapidly increased due to its very small mass; accordingly, the required stent temperature could not be maintained even if the auxiliary heating device was turned off. Therefore, the upper limit of the air pressure was 150Pa. The maximum value of the flow ratio of nitrogen to hydrogen might be 1: 2 and the air pressure could be reduced to about 40Pa; at this time, the concentration of nitrogen ions with chemical activity approximated to the maximum limit (nitrogen potential was higher). The surface current density of nitriding was high, easily resulting in uneven glow discharge of the surface of the stent. And the local stent temperature might be high, so the lower limit of the air pressure was 40Pa.

The cross section of the supporting rod of the iron-based stent obtained by using the method and the parameter range provided by the embodiment is substantially as shown in Fig. 5, which is preferably characterized in that the diffusion layer 10 accounts for 50% to 70% of the total thickness (or radius of supporting rod) and the solid solution layer 20 accounts for 15% to 25% of the total thickness (or radius of supporting rod), so that the initial corrosion rate, fatigue resistance and radial strength of the stent can be significantly optimized, thereby being favorable for reducing the risk of stent fracture caused by inward propagation of surface micro-cracks due to sudden dilatation and bending fatigue of the stent. Fig. 6 is a supporting rod of the stent observed under an electron microscope. The originally designed stent should be dilated by the balloon to an outer diameter of 3mm, but here, the stent was excessively dilated to the outer diameter of 3.6mm, so that micro-cracks were generated on the outer surface after a stress was applied to the stent, but did not directly propagate to inside of the supporting rod. A lot of slip bands generated on the outer surface of the supporting rod indicated that a typical plastic deformation occurred, and the micro-cracks only tore along the outer surface. Because of the better plasticities of the core layer 30 and the solid solution layer 20, and due to the hardness gradient and the moderate surface hardness (not more than 280HV0.01) of the diffusion layer, the surface micro-cracks did not propagate to inside of the supporting rod, and thus well reducing the risk of supporting rod fracture caused by fatigue crack propagation. That is to say, the diffusion layer 10 belonged to a higher-hardness part, and the solid solution layer 20 belonged to a lower-hardness part, the solid solution layer 20 was surrounded by the diffusion layer 10 and the two parts were separated by the interface. Based on the foregoing reasons, when the cracks in the diffusion layer 10 propagated to the solid solution layer 20, the cracks would be obstructed by the interface to stop propagating.

### Embodiment 2

According to a well-known stent grid design, the surface of a pure iron pipe was engraved by laser to be prefabricated into a pure iron vascular stent with an outer diameter of 1.6mm, an original wall thickness of 100µm, and a length of 18mm. When the stent was dilated by the balloon to 3mm, the coverage rate of a metal grid on the surface of the stent on the side wall of the stent was 13% or so. Then, the stent was treated by the following steps:

the cleaning step described in the embodiment 1 was adopted, but the current was 0.7A, the voltage was between 8V and 10V or so, and the electrochemical cleaning time was 15 seconds; thus a cleaning effect similar to the embodiment 1 was obtained. Alternatively, the current was increased to 0.9A, thereby the electrochemical cleaning time was reduced to 10 seconds and the removal amount was about 5µm (the inner and outer walls were respectively thinned by 2.5µm). Ion nitriding treatment was carried out after cleaning, and the ion nitriding treatment equipment and a part of parameters were the same as those of the embodiment 1. The parameters different from the embodiment 1 were as follows: the stent temperature of between 470°C and 500°C, the air pressure of 55Pa, and the thickness of the compound layer formed on the surface of the stent being 2µm or so.

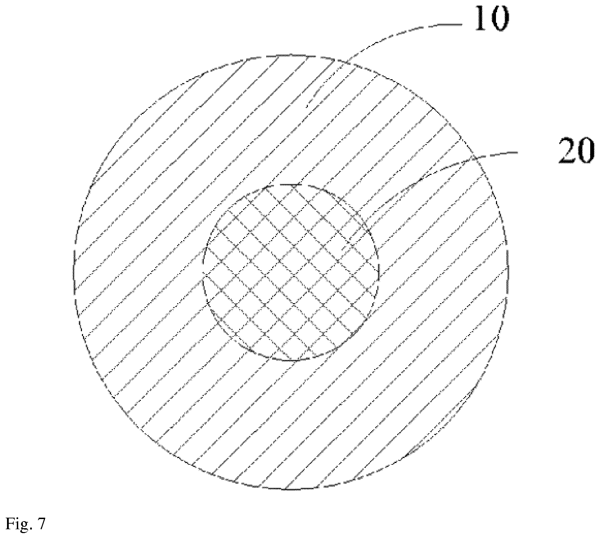

After subjecting to nitriding treatment, the stent was also subjected to polishing treatment in order to completely remove the compound layer 50. The cross section of the supporting rod of the stent was shown as in Fig. 7, and the stent surface was ensured to be smooth and clean. However, since the original wall thickness of the coronary stent was very thin, the desired surface roughness must be obtained in a lower polishing removal amount in order to ensure a certain thickness of diffusion layer 10 and solid solution layer 20. Therefore, the embodiment adopted a two-step polishing method, firstly utilized the advantage that electrochemical polishing is easy to be controlled but does not easily lead to the excessive local polishing due to its uniformity, and then utilized the advantages of low chemical polishing removal amount, high polishing efficiency, and obvious leveling and brightening effects within a short time, so that a mirror bright similar polishing effect was obtained when about 20µm of removal amount in the wall thickness direction (inner and outer walls were respectively thinned by 10µm) was removed, and the mirror bright polishing effect was obtained when the removal amount does not exceed 30µm. The first-step polishing was performed as follows: the stent was placed in an electrochemical polishing solution formed by uniformly mixing anhydrous acetic acid and perchloric acid with a mass concentration of 70% according to a volume ratio of 85:15 and was polished for 30 seconds at a temperature of between 20°C and 25°C, a current of 0.7A and a voltage of about 8 to 10V. In this process, it is better to change the electrode clamping direction of the stent so as to ensure that the wall thickness of each part of the stent is uniformly removed. Then the stent was taken out and placed in a sodium hydroxide solution with a mass ratio of 2% to 8% to be cleaned for 5 minutes, and then ultrasonically cleaned in absolute ethyl alcohol for 10 minutes. Then the second-step polishing treatment was performed, i.e., chemical polishing. Preferably, a three-acid system polishing solution using phosphoric acid, sulfuric acid and nitric acid as main components comprises 30% to 55% H₃PO₄, 20% to 45% of H₂SO₄, 5% to 15% of HNO₃ and 15% to 30% water. Phosphoric acid, sulfuric acid and nitric acid with appropriate concentration (mass percentage) were uniformly mixed according to an appropriate volume ratio to be prepared into the three-acid system polishing solution within the above-mentioned component range. In the embodiment, about 80% by mass of phosphoric acid, about 90% by mass of sulfuric acid, and about 60% by mass of nitric acid were mixed according to a volume ratio of 5: 3: 2 to be prepared into the three-acid system polishing solution. The stent was immersed in the three-acid system polishing solution and polished for 4 to 6 seconds at a temperature of 120°C (selected between 100°C and 180°C), and then the stent was taken out to be ready for cleaning. Although the nitric acid had very high volatility at higher temperatures, the composition ratio of the three-acid system polishing solution was still maintained within the above-mentioned effective range because each polishing time of the stent was very short. The better cleaning method could be used here. For example, the polished stent was cleaned with purified water and then ultrasonically cleaned in absolute ethyl alcohol for 10 minutes. Preferably, the stent was cleaned in an alkaline solution before being cleaned in the absolute ethyl alcohol, i.e., the polished stent was placed in the sodium hydroxide solution with a mass concentration of 2% to 8% to be cleaned for 5 minutes or so. In order that the surface roughness Ra reached below 0.01µm, only about 20µm of a wall thickness wasneeded to be removed by using a two-step polishing; the removal amount with the method was reduced by more than half compared with a single-step electrochemical polishing, which was very favorable for optimizing the process quality of the stent. According to the need, the first-step polishing time was set to 25 to 35 seconds, and the second-step polishing time was set to 3 to 8 seconds. The removed wall thickness could be controlled between 15µm and 25µm (including inner and outer walls) by precisely selecting appropriate two-step polishing time, or adjusting the concentration and temperature of the three-acid system polishing solution in the abovementioned range.

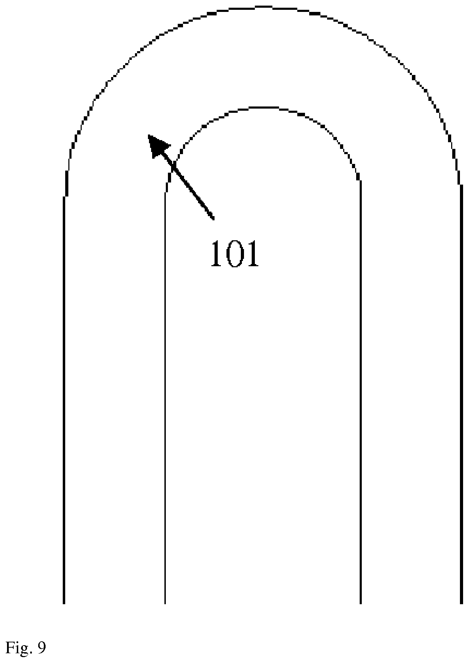

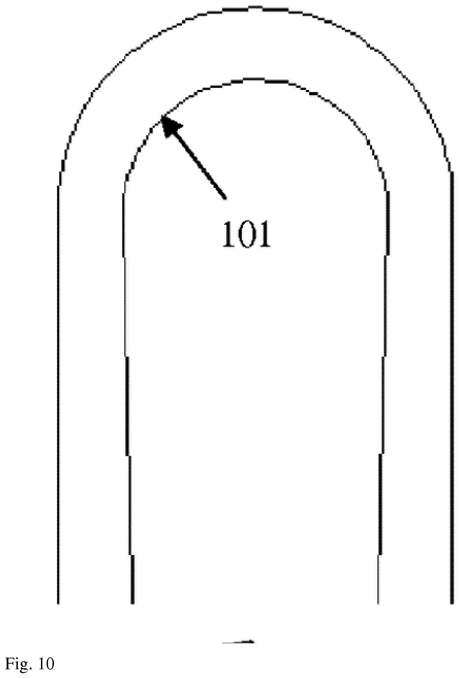

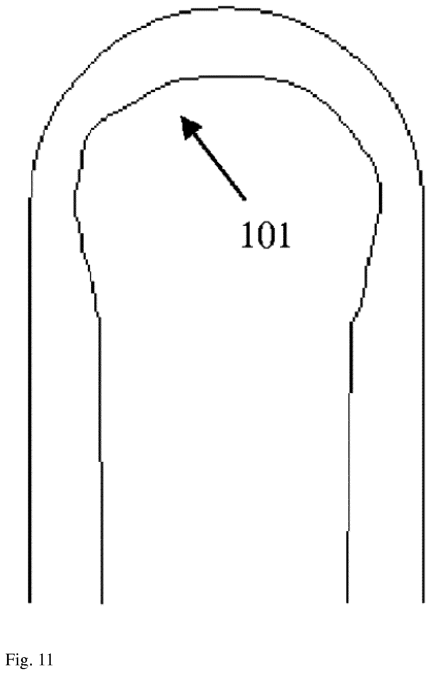

If only the chemical polishing treatment was carried out but the electrochemical polishing was omitted after nitriding, the chemical polishing time must be increased by about 2 to 3 seconds in order to remove the compound layer 10 (taking the abovementioned three-acid system polishing solution and temperature range as an example); the total removed thickness of inner and outer walls was about 15µm, but the surface roughness was slightly worse than that of the two-step polishing treatment. However, the long-duration single-step chemical polishing would result in unfavorable results as shown in Figs. 9 to 11. Generally, the engraved vascular stent contains some roundabout fine patterns. Fig. 9 is a schematic view of a section of U-shaped supporting rod of the vascular stent before polishing treatment, and a U-shaped clearance formed by a narrow bending part 101 may be as small as 200µm. The acidity obtained after anhydrous acetic acid and perchloric acid are mixed is not very strong, and the electrochemical polishing process at room temperature is relatively moderate. The clearance at the narrow bending part 101 will also remain substantially uniform isotropic corrosion even if the electrochemical polishing time is a little longer. After subjecting to continuous electrochemical polishing for 60 seconds, the supporting rod of the vascular stent became thin, but the contour of the narrow bending part 101 did not deform substantially, as shown in Fig. 10. When the electrochemical polishing time was prolonged to 150 seconds, the local deformation was not significant. Meanwhile the chemical polishing solution is a strong acid system generally and has a stronger corrosion resistance at higher temperatures.Therefore the chemical polishing process is usually intenser than the electrochemical polishing. In the chemical polishing process of heating, a nitrogen oxide gas would be rapidly generated on the metal surface, and a large number of rapidly expandable bubbles were formed in the polishing solution. A large number of bubbles were also gathered in the vicinity of the narrow bending part 101, and theses bubbles did not easily overflow from the clearance. The bubbles broke in the gaps after expanding and mutually colliding. There were a large number of bubbles which continuously broke to generate continuous dramatic impact on the narrow bending part 101, resulting in the additional impact corrosion in the gaps. When the chemical polishing time was too long, the impact corrosion caused by bubbles gathering would be severe; as a result, the narrow bending part 101 was non-homogeneously corroded. For example, after the chemical polishing was continuously carried out for 4 to 6 seconds, the narrow bending part 101 had a contour 10 as shown in Fig.10. When the chemical polishing was continuously carried out for more than 10 seconds, the narrow bending part 101 was locally and excessively corroded to be easily and severely deformed as shown in Fig. 11. The abovementioned two-step polishing treatment was adopted, i.e., the first step was the easily controlled and more moderate electrochemical polishing, and the second step was the highly efficient chemical polishing within 8 seconds. The two-step polishing treatment was more favorable for preventing local deformation of the stent caused by local excessive corrosion compared with the single-step chemical polishing, and the polishing removal amount required by reaching a mirror polishing effect was also much smaller than the removal amount of the single-step electrochemical polishing.

In the embodiment, the wall thickness of the stent was 75µm after the compound layer 50 was completely removed. It was reduced by 25µm compared with the wall thickness of an original pure iron pipe, and the mass of the stent was about 12mg. The cross section of the supporting rod of the stent (simplified as a round shape) is shown in Fig. 7, and the diffusion layer 10 and the solid solution layer 20 are sequentially arranged from outside to inside because of a surface nitriding effect under specific parameters.

The obtained diffusion layer 10 accounted for about 86.7%, the solid solution layer 20 accounted for about 13.3%, and the outer surface hardness of the diffusion layer 10 was 270HV0.01; wherein the size of diffused second-phase particles was between 30nm and 2µm. In the embodiment, the electrochemical corrosion current density of the coronary stent in the simulated body fluid PBS was about 22µA / cm², and was two times larger than 10µA / cm² of pure iron. After the coronary stent was dilated by the balloon to 3mm, the radial strength of the coronary stent was measured to be 142kPa which was increased by 31.5% compared with the radial strength of 108KPa of the pure iron stent with the same design and size. The solid solution layer 20 had a very good inhibition effect on surface micro-crack propagation, and the effect is shown in Fig. 6. The size of second-phase particles in the diffusion layer 10 was preferably between 30nm and 500nm, and could be controlled by reducing the nitrided stent temperature to about 470°C, reducing the flow ratio of hydrogen to nitrogen to 1: 5 to 1: 7 and maintaining the air pressure of about 55Pa and the bias of about 600V. At this time, the size of most of the second-phase particles was between 30nm and 500nm (the average size was also in the range), and the electrochemical corrosion current density of the surface of the stent could reach 26µA / cm² or so. If the flow ratio of hydrogen to nitrogen was reduced to 1: 5 while the bias was being increased to 650 to 750V or the air pressure was being increased to 65 to 70Pa, the stent temperature was being maintained between 470°C and 500°C and the nitriding time was being maintained for 30 minutes, the nitriding current density was still maintained within a range of 0.5 to 0.8 mA/cm²; accordingly, a structure and a property effect similar to Fig. 7 were obtained. Alternatively, if the flow ratio of hydrogen to nitrogen was reduced to 1: 7 while the bias was being increased to 750 to 800V or the air pressure was being increased to 85 to 100Pa, a structure and a property effect similar to Fig. 7 were also obtained. Based on the method provided by the embodiment, the cross section of the supporting rod as shown in Fig. 7 was obtained; wherein the solid solution layer 20 preferably accounted for 10% to 25% of the total thickness (or radius of supporting rod), and the diffusion layer 10 accounted for 75% to 90% of the total thickness; thus the initial corrosion rate, fatigue resistance and radial strength of the stent could be obviously optimized, especially the former two properties.

### Embodiment 3

The corresponding steps described in the embodiment 1 were adopted to produce the same pure iron vascular stent, and the pure iron vascular stent was subjected to ion nitriding treatment after being cleaned. In the embodiment, the ion nitriding treatment equipment and a part of parameters were the same as those of the embodiment 1, and the parameters different from the embodiment 1 were set as follows: the nitriding temperature of 470 to 500 °C was maintained, the nitriding time was 60 minutes, and the thickness of the compound layer 50 formed on the surface of the stent was 3 to 4µm. After subjecting to nitriding treatment, the stent was also subjected to polishing treatment in order to completely remove the compound layer 50, and ensure that the stent surface was smooth and clean. The polishing treatment step described in the embodiment 1 was adopted, but the polishing time was prolonged by 10 seconds so as to uniformly remove the thickness of about 50µm of the stent, and the surface roughness also reached 0.01µm below.

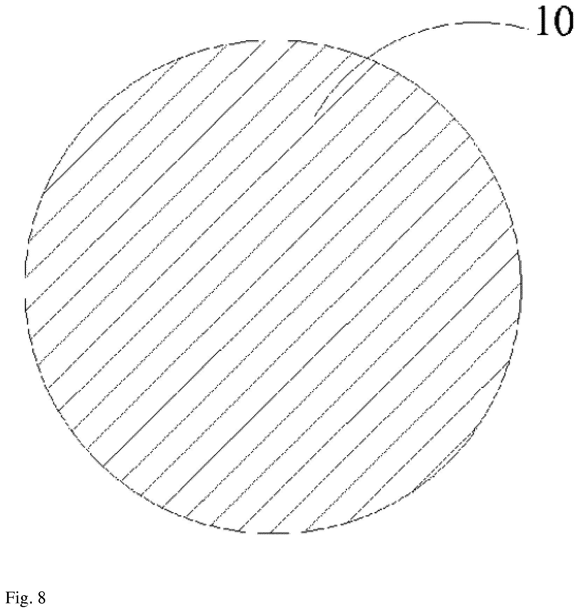

In the embodiment, the wall thickness of the stent after cleaning, nitriding and polishing to completely remove the compound layer 50 was 165µm, and was reduced by 55µm compared with the wall thickness of the original pure iron pipe.The cross section (simplified as a round shape) of the supporting rod of the stent was shown in Fig. 8. The continuous nitriding time was longer than that of the embodiment 1, a large number of nitrogen atoms were diffused to reach a deepest part inside the iron substrate, the iron substrate all became nitrogen-containing saturated solid solutions, the second phase was precipitated from the saturated solid solutions by excessive nitrogen atoms, and the second-phase particles had been diffused to all regions of the saturated solid solutions; therefore, only the diffusion layer 10 was left on the cross section of the supporting rod. The surface micro-hardness of the diffusion layer 10 obtained in the embodiment was about 270HV0.01. Meanwhile, there were different nitrogen atom concentrations at different depths of the diffusion layer 10 from the surface, the nitrogen atom concentrations from outside to inside were gradually decreased, the central region of the diffusion layer 10 had a minimum concentration value of nitrogen atoms and thus the micro-hardness here was about 230HV0.01; that was still increased by 60HV0.01 compared with the micro-hardness of the pure iron pipe. In view of the diffusion layer 10 having a higher hardness, the radial strength of the stent was measured to be about 65kPa after the stent in the embodiment was dilated by the balloon to 12mm , and the radial strength of the pure iron stent with the same shape and size (165µm wall thickness) was 40kPa; thus the radial strength was increased by more than 60%. The region, nearby the surface, of the diffusion layer 10 had the characteristics of a high hardness and a poor plasticity, and the central region of the diffusion layer 10 had a slightly low hardness but has a better plasticity (slightly worse than a pure iron material); therefore, the stent provided by the embodiment still had a better fatigue resistance; that is, inhibition effects on micro-crack initiation and inward micro-crack propagation has been clearly described in the embodiment 1. In the embodiment, the size of second-phase particles in the diffusion layer 10 was between 30nm and 2µm, and the electrochemical corrosion current density of the diffusion layer 10 in the simulated body fluid PBS was about 15µA / cm² which was increased by about 50% compared with 10µA / cm² of pure iron. Preferably, when the nitrided stent temperature was reduced to 470°C and the flow ratio of nitrogen to hydrogen was reduced to 1: 5 to 1: 7, the size of second-phase particles was basically in the preferable range of 30nm to 500nm. The galvanic corrosion of the diffusion layer 10 in the in-vivo environment could be obviously accelerated in the preferable range, and the electrochemical corrosion current density of the diffusion layer 10 in the simulated body fluid PBS was about 18µA / cm².

In the embodiment, the whole stent was the diffusion layer 10; that is to say, each part (including an inner part) of the stent had a higher nitrogen content, and the hardness of inside of the supporting rod was greater than that of the supporting rod in the embodiment 1; accordingly the overall strength of the stent was greatly improved, and the stent had the advantage of easiness in galvanic corrosion on the whole. In summary, the embodiment was most favorable for improving the radial strength of the stent and shortening the time required by absorbing the stent; meanwhile, the fatigue resistance and smaller wall thickness were also ensured, and the stent had better comprehensive properties.

### Embodiment 4

The corresponding steps described in the embodiment 2 were adopted to produce the same pure iron vascular stent, and the pure iron vascular stent was subjected to ion nitriding treatment after being cleaned. In the embodiment, the ion nitriding treatment equipment and a part of parameters were the same as those of the embodiment 2, and the parameters different from the embodiment 1 were set as follows: the nitriding temperature of 450 to 480 °C was maintained, the nitriding time was 60 minutes, and the thickness of the compound layer 50 formed on the surface of the stent was 3 to 4µm.

After subjecting to nitriding treatment, the stent was also subjected to polishing treatment in order to completely remove the compound layer 50, and ensure that the stent surface was smooth and clean. The two-step polishing treatment step described in the embodiment 2 was adopted. In view of the electrochemical polishing treatment time being easier to be precisely controlled, the electrochemical polishing time was just prolonged by 15 seconds so as to uniformly remove the thickness of about 25µm of the stent, and the surface roughness also reached 0.01µm below.

In the embodiment, the wall thickness of the stent after cleaning, nitriding and polishing was 70µm, and was reduced by 30µm compared with the wall thickness of the original pure iron pipe. The cross section (simplified as a round shape) of the supporting rod of the stent is shown in Fig. 8. The continuous nitriding time was longer than that of the embodiment 2, a large number of nitrogen atoms reached a deepest part inside the iron substrate, all of the iron substrate became nitrogen-containing saturated solid solutions, and the second-phase particles had been diffused to all regions of the saturated solid solutions; therefore, only the diffusion layer 10 was left on the cross section of the supporting rod. The diffusion layer 10 obtained by the embodiment had the following characteristics that: the deepest parts of the diffusion layer 10 from the surface had nitrogen atom concentration gradients, the surface Vickers micro-hardness of the diffusion layer 10 was 280HV0.01 or so, the central region of the diffusion layer 10 had a minimum concentration of nitrogen atoms and the micro-hardness here was about 230HV0.01 (still increased by 60HV0.01 compared with the micro-hardness of the pure iron pipe). Therefore, the stent in the embodiment still had a better fatigue resistance, i.e., inhibition effects on surface micro-crack initiation and inward micro-crack propagation, which had been clearly described in the abovementioned embodiments. The radial strength of the stent was measured to be 140kPa after the stent was dilated by the balloon to 3mm and was increased by 73.9% compared with the radial strength of 92kPa of the pure iron stent with the same shape and size (70µm wall thickness). The size of second-phase particles in the diffusion layer of the stent was between 30nm and 2µm, and the electrochemical corrosion current density of the stent with the characteristic structure in the simulated body fluid PBS was 15µA / cm² or so which was increased by about 50% compared with 10µA / cm² of pure iron. The size of second-phase particles could be controlled in the preferable range of 30nm to 500nm by reducing the nitrided stent temperature to 450°C and reducing the flow ratio of nitrogen to hydrogen to 1: 5 to 1: 7; at the moment, the electrochemical corrosion current density of the stent in the simulated body fluid PBS could be increased to 18µA / cm² or so. In the embodiment, under the premise that the temperature was maintained between 450°C and 480°C and the nitriding time was 60 minutes, the flow ratio of nitrogen to hydrogen could be reduced to 1: 5 while the bias was being increased to 700 to 750V or the air pressure was being increased to 70 to 75Pa; the flow ratio of nitrogen to hydrogen could also be reduced to 1: 7 while the bias was being increased to 750 to 800V or the air pressure was being increased to 90 to 100Pa; at the moment, the nitriding current density was still maintained in a range of between 0.5mA / cm² and 0.8mA / cm²; accordingly, a structure and a property effect similar to those obtained after the abovementioned nitriding treatment could be still obtained.

In summary, the embodiment was more favorable for improving the radial strength of the stent and shorting the time required by absorbing the stent compared with the embodiment 2. Meanwhile it also ensured the fatigue resistance and the smaller wall thickness and had better comprehensive properties.

### Embodiment 5

The corresponding steps described in the embodiment 1 were adopted to produce the same pure iron vascular stent, and the pure iron vascular stent was subjected to ion nitriding treatment after being cleaned. In the embodiment, the ion nitriding treatment equipment and a part of parameters were the same as those of the embodiment 1, and the parameters different from the embodiment 1 were set as follows: the nitriding stent temperature of 450 to 470 °C was maintained, the nitriding time was 120 minutes, and the thickness of the compound layer 50 formed on the surface of the stent was about 5µm.

After subjecting to nitriding treatment, the stent was also subjected to polishing treatment in order to completely remove the compound layer 50, and to ensure that the stent surface was smooth and clean. The polishing treatment step described in the embodiment 1 was adopted, but the polishing time was prolonged by 20 seconds so as to uniformly remove the thickness of about 55µm of the stent. The surface roughness Ra of the stent also reached 0.01µm below.

In the embodiment, the wall thickness of the stent after cleaning, nitriding and polishing was 160µm, which was reduced by 60µm compared with the wall thickness of the original pure iron pipe.The cross section (simplified as a round shape) of the supporting rod of the stent is shown in Fig. 8. Because the nitriding process was thoroughly carried out, a large number of nitrogen atoms reached the deepest part inside the iron substrate. The concentrations of nitrogen atoms in the diffusion layer 10 were basically uniform, and the second-phase particles had been more uniformly distributed in all regions of the diffusion layer 10; that is to say, only the more uniform diffusion layer 10 was left on the cross section of the supporting rod. The micro-hardness of each region of the diffusion layer 10 obtained by the embodiment was basically in a range of between 280HV0.01 and 310 HV0.01, which has a very good inhibition effect on micro-crack initiation. In the embodiment, the radial strength of the obtained stent was measured to be 65kPa after the stent was dilated by the balloon to 12mm , which was increased by more than 80% compared with the radial strength of 35kPa of the pure iron stent with the same shape and size (160µm wall thickness). The size of second-phase particles in the diffusion layer 10 obtained under the nitriding process conditions was basically in a range of 30nm to 2µm; however, because of 2-hour long-time nitriding, the nitrogen content of the diffusion layer 10 was high (weight percentage of 2.7% to 3.7%), the hardness was high, the second phase agglomerated and grew, and the size concentrated in a range of 1µm and 2µm; thus the cathode area of micro galvanic corrosion was reduced; meanwhile, the long-time nitriding played an annealing role in the stent to a certain extent; the distorted and disordered grain boundaries, high residual stress and dislocation density which were originally brought by cold working such as drawing were reduced to a certain extent or even completely restored, and thus the effect that the corrosion rate was accelerated by original tissues was eliminated; therefore the corrosion rate was not more than that of the diffusion layer 10 with a hardness value of 280HV0.01 instead. The electrochemical corrosion current density of the stent in the simulated body fluid PBS could be experimentally measured to be about 12µA / cm² which was slightly increased compared with 10µA / cm² of pure iron. Since the whole stent was the more uniform diffusion layer 10, and the diffusion layer 10 was easier to be subjected to galvanic corrosion than the non-nitrided pure iron; therefore, the overall corrosion rate of the stent could be improved. In summary, under the premise that the wall thickness was reduced as much as possible, the embodiment was most favorable for improving the radial strength of the stent, and shortening the time required by absorbing the stent to a certain extent.

### Embodiment 6

The corresponding steps described in the embodiment 2 were adopted to produce the same pure iron vascular stent, and the pure iron vascular stent was subjected to ion nitriding treatment after being cleaned. In the embodiment, the ion nitriding treatment equipment and a part of parameters were the same as those of the embodiment 2, and the parameters different from the embodiment 2 were set as follows: the nitriding stent temperature of 430 to 450 °C was maintained, the nitriding time was 120 minutes, and the thickness of the compound layer 50 formed on the surface of the stent was about 5µm.

After subjecting to nitriding treatment, the stent was also subjected to polishing treatment in order to completely remove the compound layer 50 and to ensure that the stent surface was smooth and clean. The two-step polishing method described in the embodiment 2 was adopted, but the polishing time was prolonged by 30 seconds so as to uniformly remove the thickness of about 30µm of the stent; the surface roughness Ra of the stent also reached 0.01µm below.

In the embodiment, the wall thickness of the stent after cleaning, nitriding and polishing was 65µm which was reduced by 35µm compared with the wall thickness of the original pure iron pipe. The cross section (simplified as a round shape) of the supporting rod of the stent is shown in Fig. 8. Because the nitriding process was thoroughly carried out, a large number of nitrogen atoms reached the deepest part inside the iron substrate.The concentrations of nitrogen atoms in the diffusion layer 10 were basically uniform, and the second-phase particles had been more uniformly distributed in all regions of the diffusion layer 10; that is to say, only the diffusion layer 10 was left on the cross section of the supporting rod. The micro-hardness of each region of the diffusion layer 10 obtained in the embodiment was basically in a range of between 290HV0.01 and 320 HV0.01, which had a very good inhibition effect on micro-crack initiation. In the embodiment, the radial strength of the obtained stent was measured to be 135kPa after the stent was dilated by the balloon to 3mm, which was increased by 100% compared with the radial strength of 68kPa of the pure iron stent with the same shape and size (65µm wall thickness). The size of second-phase particles in the diffusion layer 10 obtained under such nitriding process conditions was basically in a range of 30nm to 2µm, and the corrosion rate was less than that of the diffusion layer 10 with a hardness value of 280HV0.01 below, which had been clearly described in the embodiment 5. The electrochemical corrosion current density of the stent in the simulated body fluid PBS was experimentally measured to be 12µA / cm² or so, which was slightly increased compared with 10µA / cm² of pure iron. Similarly, the preferable process in the embodiment 4 could be adopted, accordingly, the size of second-phase particles was basically in a preferable range of 30nm to 500nm and the electrochemical corrosion current density of the obtained diffusion layer 10 could be increased to about 15µA / cm². Since the whole stent was the more uniform diffusion layer 10 and the diffusion layer 10 was easier to be subjected to galvanic corrosion than the non-nitrided pure iron, the overall corrosion rate of the stent could be improved. In summary, under the premise that the wall thickness was reduced as much as possible, the embodiment was most favorable for improving the radial strength of the stent, and shortened the time required by absorbing the stent to a certain extent, and was suitable for a thin-walled absorbable coronary stent requiring very high radial strength.

Advantages of the present invention. The iron-based implantable vascular stent (e.g., a supporting rod of a stent) after nitriding may have a cross section as shown in one of Fig. 5, Fig. 7 and Fig.8; wherein a layered structure of the device may have the preferred relative thickness (measured from the center to outside), thus an effect that the outer part is hard and the inner part is rough is most favorably generated. The diffusion layer 10 with a higher hardness helps to improve the structural strength, and the core layer 30 and the solid solution layer 20 which have very good plasticities can improve the fatigue resistance. The method of the present invention can control the concentration distribution and diffusion degree of nitrogen atoms in the iron substrate, and realizes the preferable thicknesses of the diffusion layer 10 and the solid solution layer 20, so that a certain thickness of solid solution layer 20 even the core layer 30 is reserved inside the supporting rod of the stent for improving the plasticity of the supporting rod of the stent. Furthermore, under the premise of not reducing the radial strength of the stent, the nitrided stent can have a smaller wall thickness, such as a coronary stent wall thickness of 60 to 75µm, a smaller peripheral stent wall thickness of 120 to 130µm, and a larger peripheral stent wall thickness of 160 to 170µm. The thin-walled stent can not only improve flexibility and greatly improve the bending property in complex lesion blood vessels, but also shorten the entire corrosion cycle of the bioabsorbable stent, reduce the metal amount of the stent and further reduce the local accumulation of iron ions in tissues; the total mass of such a stent is 5 to 100mg or so. The maximum wall thickness of the vascular stent can reach 300µm. Both the mechanical properties and the corrosion rate of thick-walled stent can be considered and optimized in the present invention.

Several multi-layer structures provided by the present invention can be applied to a coronary stent, a peripheral stent and a non-vascular stent, or other implantable medical devices containing absorbable components. The original pipe material selected is pure iron or iron alloys containing more than 99% by mass of iron. Based on the treatment method provided by the present invention, the thickness of the pure iron or iron alloy prefabricated component should be 10 to 100µm larger than the corresponding thickness of the corresponding component of the product. The thickness of the prefabricated component is more than 10µm larger than the thickness of the absorbable component. Since the compound layer 50 is formed on the surface of the prefabricated component during nitriding, the nitrided prefabricated component is subjected to electrochemical or chemical polishing treatment; at least 5µm thick surface (including the whole compound layer 50 and a small amount of diffusion layer 10) of the prefabricated component should be removed, and the thickness is generally reduced by 20 to 60µm.

The present invention focuses on optimization of a multi-layer structure property of an absorbable vascular stent and a preparation method thereof. Under the premise of ensuring the radial strength of the stent, the wall thickness of the iron-based stent is reduced, the corrosion rate and flexibility of the stent are improved, and the multi-layer structure has wider adaptability. Because interventional medical devices, especially stent products, have two conflicting factors of flexibility and strength, both the flexibility and the strength should be reasonably taken into consideration according to the characteristics and treatment requirements of lesion parts. A vascular stent is taken as an example: in practical clinical application, the lesion blood vessel situations are complex, and have different requirements for the flexibility and the radial strength of the vascular stent. A severely calcified lesion vascular segment requires a higher-radial-strength stent; however, a higher-flexibility stent will be more suitable for a complex bending vascular segment. Meanwhile, for an absorbable iron-based medical device, the corrosion degradation property is also one of the most critical properties. The corrosion degradation property and other properties need be mutually reconciled so that the comprehensive property of the medical device is more targetedly adapted to a certain particular clinical symptom; therefore the present invention provides the multi-layer structures of the absorbable iron-based medical device, which can meet different application needs, as shown in Fig. 5, Fig. 7 or Fig. 8.

The present invention provides a treatment method for subjecting a bioabsorbable iron-based vascular stent to ion nitriding and polishing treatment. In order that the nitrided compound layer extends to inside of the iron-based material in a dendritic or flaky shape, a lower stent temperature can be selected during nitriding, such as below 550°C. In order to prevent generation of a thicker compound layer, the bias is preferably above 600V during nitriding. Different internal material structures of the iron-based device can be obtained by different implementation methods of surface nitriding treatment steps. A stent is taken as an example:the nitriding process parameters are adjusted in the abovementioned range, then the stent is subjected to the subsequent treatment step, the cross section of the supporting rod of the stent has a structure shown in one of Fig. 5, Fig. 7 and Fig. 8, and the proportion of each layer reaches an optimized range; wherein the preferable size of the second-phase particles in the diffusion layer 10 is between 30nm and 500nm, the micro-hardness of the diffusion layer 10 is in a preferably range of 220 to 320HV0.01, and the nitrogen content of the diffusion layer is preferably 1.0 to 3.7 wt-%, so that the galvanic corrosion of the absorbable iron-based component in the in vivo environment can be significantly speeded up and the electrochemical corrosion rate is two times higher than that of a pure iron material before being treated by the method of the present invention.

In the prior art, the electrochemical polishing adopted after nitriding has significant disadvantages that a more smooth and bright surface can only be obtained when the removal amount of the wall thickness of the stent reaches 40µm, and the polished surface roughness can only be controlled below 0.1µm. In order to improve the effect of the polishing treatment, an improvement on the polishing process after pure iron nitriding was performed by the present invention, and the two-step polishing step was given for a thin-walled stent (original wall thickness≤100µm); accordingly, when the removal amount of the wall thickness of the supporting rod reached 20µm, the 0.01µm below surface roughness of inner and outer surfaces of the stent could be realized to obtain a mirror bright similar effect,

Compared with the prior art, the bioabsorbable vascular stent provided by the present invention has the following advantages:.
1. the absorbable component in the bioabsorbable medical device is internally provided with a multi-layer structure; an absorbable stent was taken as an example, in which the mutually contradictory characteristics of corrosion rate, radial strength, flexibility, and wall thickness of the stent could achieve a relatively perfect balance point; the technical solution of the present invention also provides a preparation method of the absorbable vascular stent, and determines preferred process parameters and corresponding property effects;
2. by using preferable mixed gas flow ratio, air pressure, component temperature, bias, nitriding time and component temperature, the internal structure of the component can be controlled, and the thickness of the compound layer on the surface of the component is below 5µm; the situation that the compound layer is completely removed is ensured through preferable electrochemical or chemical polishing treatment; both the inner and outer surfaces of the component achieve a mirror bright effect in a low polishing removal amount, and finally, the absorbable components of different internal structures can be obtained;
3. the optimized nitriding process and polishing process provided by the present invention can ensure that under the conditions of same stent grid design and wall thickness, the supporting force of the absorbable iron-based vascular stent (the stent wall thickness can be reduced to 60µm) is comparable with a cobalt-chromium alloy stent; on coronary stents and peripheral stents, such an effect can be obtained; the radial strength of the stent is increased by 31.5% to 94.4% compared with a pure iron stent;
4. the present invention provides a bioabsorbable iron-based vascular stent ; it comprises a multi-layer structure having a gradient property, including the diffusion layer, the solid solution layer and the core layer; the supporting force and flexibility of the stent can be simultaneously improved, and the corrosion resistance is reduced; the incidence of fatigue cracks in the surface can be reduced, and the propagation of surface microcracks to the inside of metal can also be inhibited, so that the absorbable medical device has a higher fatigue fracture resistant property in the early period when the absorbable medical device is implanted in the human body; after the pure iron component is subjected to surface nitriding, it has a high strength and a high hardness because of the strengthening effect of second-phase particles in the diffusion layer, so that the supporting property of the stent can be effectively improved; the solid solution layer and the core layer keep a higher elongation and a good plasticity similar to pure iron; because most of the fatigue cracks are firstly formed in the metal surface, the fatigue crack initiation can be prevented by improving the component surface hardness and refining grains; on the other hand, the improvement on the plasticity of inside of the component will help to inhibit crack propagation; therefore, the propagation of cracks to inside of the component can be effectively inhibited by reserving the solid solution layer or core layer with a lower hardness inside the component;
5. the corrosion rate of the bioabsorbable vascular stent can be increased by two times or so compared with a pure iron medical device, and the corrosion rate of the bioabsorbable vascular stent in the simulated body fluid PBS can be increased by 180% compared with pure iron; when the outer surface hardness of the diffusion layer is controlled between 230 HV0.01 and 270HV0.01, the finer the second-phase particles in the diffusion layer are, the faster the corrosion rate is; due to the presence of a certain number of second-phase particles in the diffusion layer, the material is easily subjected to galvanic corrosion when being subjected to normal corrosion, thereby shortening the time required by human body to absorb the medical device.

## Claims

1. A bioabsorbable vascular stent, comprising an absorbable component produced by subjecting a prefabricated component made from an iron-based raw material to ion nitriding treatment, the material composition inside the absorbable component changing with the depth from the surface, and
wherein the absorbable component comprises at least a first part and a second part, the second part being surrounded by the first part and the hardness of the first part is higher than that of the second part,
**characterized in that** the first part comprises a dispersion layer (10) which is continuously distributed inwardly from its surface, but does not include a corrosion resistant layer (50) formed of γ'-phase and ε-phase nitrides on its surface, the dispersion layer (10) being a solid solution with a nitrogen content of less than 1 wt-% and particles with a nitrogen content of more than 1wt-% being scattered in the solid solution,
the second part comprises a solid solution layer (20) surrounded by the dispersion layer (10), such that the first part includes the whole dispersion layer (10) and the second part includes the whole solid solution layer (20),
wherein the corrosion resistant layer (50) is completely removed from the component, and
wherein the dispersion layer (10) and the solid solution layer (20) are respectively positioned on either side of an interface constituted to inhibit cracks generated in the dispersion layer (10) from extending into the solid solution layer (20).

2. The bioabsorbable vascular stent as set forth in claim 1, **characterized in that** the nitrogen wt-% in the dispersion layer (10) is between 1% and 3.7%.

3. The bioabsorbable vascular stent as set forth in claim 1, **characterized in that** the particles account for less than 63 wt-% of the dispersion layer (10).

4. The bioabsorbable vascular stent as set forth in claim 3, **characterized in that** sizes of most particles are between 30nm and 2µm, and the part having the maximum hardness of the absorbable component is positioned nearby the surface of the absorbable component and the maximum Vickers hardness is not more than 350HV0.01; the hardness of each part of the dispersion layer (10) is increased by 50HV0.01 compared with the Vickers hardness of non-nitrided iron-based raw material of the prefabricated component.

5. The bioabsorbable vascular stent as set forth in claim 4, **characterized in that** the Vickers hardness of the dispersion layer (10) is more than 220HV0.01 and less than 320HV0.01 and gradually decreases with the depth.

6. The bioabsorbable vascular stent as set forth in claim 1, **characterized in that** the absorbable component comprises a plurality of struts connected to form a tubular grid; the vascular stent is produced by subjecting an iron-based pipe with a uniform wall thickness to engraving and ion nitriding treatment, and the wall thickness of the vascular stent is between 60µm and 300µm; under the conditions of the same tubular grid and wall thickness, the radial strength of the vascular stent is increased by more than 30% compared with the radial strength of the non-nitrided vascular stent prefabricated by the iron-based raw material.

7. The bioabsorbable vascular stent as set forth in claim 6, **characterized in that** the vascular stent has a wall thickness of between 60µm and 300µm and a mass between 5mg and 100mg, in particular a wall thickness between 60µm and 100µm or a thickness between 160µm and 170µm and
wherein a Vickers hardness of the vascular stent is between 280HV0.01 and 320HV0.01; under the conditions of the same tubular grid and wall thickness, the radial strength of the vascular stent is increased by more than 80% compared with the radial strength of the non-nitrided vascular stent prefabricated by the iron-based raw material.

8. A method for preparing the bioabsorbable vascular of claims 1-7, comprising the following steps of:
step 1. processing a prefabricated component by using pure iron or an iron alloy containing more than 99 wt-% of iron as a raw material, an initial thickness of the prefabricated component being 10µm larger than a corresponding thickness of the vascular stent;
step 2. cleaning to remove contaminants on a surface of the prefabricated component;
step 3. treating the prefabricated component in step 2 by adopting an ion nitriding method, the temperature of the prefabricated component being between 430°C and 550°C, and the nitriding time being between 10 minutes and 200 minutes;
wherein during the nitriding step 3, a nitrogen-rich corrosion resistant layer (50) is formed on the surface of the component, and
step 4. subjecting a nitrided prefabricated component to an electrochemical polishing treatment and/or a chemical polishing treatment to completely remove the corrosion resistant layer (50) of the prefabricated component.

9. The preparation method as set forth in claim 8, **characterized in that** in step 1, a surface Vickers hardness of the prefabricated component is between 165 HV0.01 and 175HV0.01.

10. The preparation method as set forth in claim 8, **characterized in that** in step 1, a thickness of the prefabricated component is 20 to 60µm larger than a corresponding thickness of the final vascular stent.

11. The preparation method as set forth in claim 8, **characterized in that** in step 3, the temperature of the prefabricated component is between 470°C and 520°C and in step 3, the ion nitriding method adopts a mixed gas of nitrogen and hydrogen, and a flow ratio of nitrogen to hydrogen is between 1: 2 and 1: 9, and gas discharge is maintained at a gas pressure of between 40Pa and 150Pa and a bias of between 600V and 800V.

12. The preparation method as set forth in claim 8, **characterized in that** in step 4, after the electrochemical polishing treatment, the chemical polishing treatment is performed to remove 15 to 25µm thickness of the outer layer of the prefabricated component.

13. The preparation method as set forth in claim 12, **characterized in that** in step 4, a three-acid system polishing solution with a temperature of between 100°C and 180°C is adopted in the chemical polishing treatment, and the three-acid system polishing solution comprises the following main components: 30 % to 55% of H₃PO₄, 20% to 45% of H₂SO₄, 5% to 15% of HNO₃ and 15% to 30% of water.

14. The preparation method as set forth in claim 13, **characterized in that** in step 4, the chemical polishing treatment time is between 3 seconds and 8 seconds.

## Patentansprüche

1. Bioabsorbierbarer Gefäßstent, mit einer absorbierbaren Komponente, die hergestellt wird, indem eine vorgefertigte Komponente aus einem Ausgangsmaterial auf Eisenbasis einer Plasmanitrierbehandlung unterzogen wird, wobei sich die Materialzusammensetzung im Inneren der absorbierbaren Komponente von der Oberfläche aus mit der Tiefe ändert, und
wobei die absorbierbare Komponente wenigstens einen ersten Teil und einen zweiten Teil umfasst, wobei der zweite Teil vom ersten Teil umgeben ist und die Härte des ersten Teils höher als die des zweiten Teils ist,
**dadurch gekennzeichnet, dass** der erste Teil eine Dispersionsschicht (10) umfasst, die von ihrer Oberfläche kontinuierlich nach innen verteilt ist, aber keine korrosionsbeständige Schicht (50) aufweist, die aus γ'-Phasen- und ε-Phasen-Nitriden auf ihrer Oberfläche gebildet ist, wobei die Dispersionsschicht (10) ein Mischkristall mit einem Stickstoffgehalt von weniger als 1 Gew.-% ist und Teilchen mit einem Stickstoffgehalt von mehr als 1 Gew.-% in dem Mischkristall gestreut sind,
der zweite Teil eine Mischkristallschicht (20) umfasst, die von der Dispersionsschicht (10) so umgeben ist, dass der erste Teil die gesamte Dispersionsschicht (10) und der zweite Teil die gesamte Mischkristallschicht (20) aufweist,
wobei die korrosionsbeständige Schicht (50) vollständig von der Komponente entfernt ist und
wobei die Dispersionsschicht (10) und die Mischkristallschicht (20) auf jeweils einer der beiden Seiten einer Grenzfläche angeordnet sind, die so ausgebildet ist, dass sie in der Dispersionsschicht (10) erzeugte Risse daran hindert, sich in die Mischkristallschicht (20) hinein auszudehnen.

2. Bioabsorbierbarer Gefäßstent nach Anspruch 1, **dadurch gekennzeichnet, dass** der Gewichtsprozentsatz an Stickstoff in der Dispersionsschicht (10) zwischen 1% und 3,7% beträgt.

3. Bioabsorbierbarer Gefäßstent nach Anspruch 1, **dadurch gekennzeichnet, dass** weniger als 63 Gew.-% der Dispersionsschicht (10) auf die Teilchen entfallen.

4. Bioabsorbierbarer Gefäßstent nach Anspruch 3, **dadurch gekennzeichnet, dass** die meisten Teilchen zwischen 30 nm und 2 µm groß sind und der Teil mit der maximalen Härte der absorbierbaren Komponente nahe der Oberfläche der absorbierbaren Komponente gelegen ist und die maximale Vickers-Härte nicht mehr als 350HV0,01 beträgt, wobei die Härte jedes Teils der Dispersionsschicht (10) verglichen mit der Vickers-Härte eines nicht nitrierten Ausgangsmaterials auf Eisenbasis der vorgefertigten Komponente um 50HV0,01 erhöht ist.

5. Bioabsorbierbarer Gefäßstent nach Anspruch 4, **dadurch gekennzeichnet, dass** die Vickers-Härte der Dispersionsschicht (10) mehr als 220HV0,01 und weniger als 320HV0,01 beträgt und mit der Tiefe allmählich abnimmt.

6. Bioabsorbierbarer Gefäßstent nach Anspruch 1, **dadurch gekennzeichnet, dass** die absorbierbare Komponente mehrere Streben umfasst, die zu einem rohrförmigen Gitter verbunden sind, der Gefäßstent hergestellt wird, indem ein Rohr auf Eisenbasis mit einer gleichmäßigen Wandstärke einer Gravier- und Plasmanitrierbehandlung unterzogen wird, und die Wandstärke des Gefäßstents zwischen 60 µm und 300 µm beträgt, die radiale Festigkeit des Gefäßstents unter den Bedingungen des gleichen rohrförmigen Gitters und der gleichen Wandstärke verglichen mit der radialen Festigkeit des durch das Ausgangsmaterial auf Eisenbasis vorgefertigten, nicht nitrierten Gefäßstents um mehr als 30% erhöht ist.

7. Bioabsorbierbarer Gefäßstent nach Anspruch 6, **dadurch gekennzeichnet, dass** der Gefäßstent eine Wandstärke zwischen 60 µm und 300 µm und eine Masse zwischen 5 mg und 100 mg aufweist, insbesondere eine Wandstärke zwischen 60 µm und 100 µm oder eine Stärke zwischen 160 µm und 170 µm, und
wobei eine Vickers-Härte des Gefäßstents zwischen 280HV0,01 und 320HV0,01 beträgt, die radiale Festigkeit des Gefäßstents unter den Bedingungen des gleichen rohrförmigen Gitters und der gleichen Wandstärke verglichen mit der radialen Festigkeit des durch das Ausgangsmaterial auf Eisenbasis vorgefertigten, nicht nitrierten Gefäßstents um mehr als 80% erhöht ist.

8. Verfahren zur Herstellung des bioabsorbierbaren Gefäßstents nach den Ansprüchen 1-7, mit den folgenden Schritten:
Schritt 1. Verarbeiten einer vorgefertigten Komponente unter Verwendung von reinem Eisen oder einer Eisenlegierung, die mehr als 99 Gew.-% Eisen als Ausgangsmaterial enthält, wobei eine anfängliche Dicke der vorgefertigten Komponente 10 µm größer als eine entsprechende Dicke des Gefäßstents ist;
Schritt 2. Reinigen zum Entfernen von Verunreinigungen auf einer Oberfläche der vorgefertigten Komponente;
Schritt 3. Behandeln der vorgefertigten Komponente in Schritt 2 durch Anwenden eines Plasmanitrierverfahrens, wobei die Temperatur der vorgefertigten Komponente zwischen 430°C und 550°C liegt und die Nitrierzeit zwischen 10 Minuten und 200 Minuten beträgt;
wobei während des Nitrierschritts 3 eine stickstoffreiche, korrosionsbeständige Schicht (50) auf der Oberfläche der Komponente gebildet wird, und
Schritt 4. Eine nitrierte vorgefertigte Komponente wird einer elektrochemischen Polierbehandlung und/oder einer chemischen Polierbehandlung unterzogen, um die korrosionsbeständige Schicht (50) der vorgefertigten Komponente vollständig zu entfernen.

9. Herstellungsverfahren nach Anspruch 8, **dadurch gekennzeichnet, dass** in Schritt 1 eine Oberflächenhärte nach Vickers der vorgefertigten Komponente zwischen 165 HV0,01 und 175HV0,01 liegt.

10. Herstellungsverfahren nach Anspruch 8, **dadurch gekennzeichnet, dass** in Schritt 1 eine Dicke der vorgefertigten Komponente 20 bis 60 µm größer als eine entsprechende Dicke des endgültigen Gefäßstents ist.

11. Herstellungsverfahren nach Anspruch 8, **dadurch gekennzeichnet, dass** in Schritt 3 die Temperatur der vorgefertigten Komponente zwischen 470°C und 520°C liegt und in Schritt 3 bei dem Plasmanitrierverfahren ein Mischgas aus Stickstoff und Wasserstoff angewendet wird, und ein Strömungsverhältnis von Stickstoff zu Wasserstoff zwischen 1:2 und 1:9 liegt und die Gasentladung bei einem Gasdruck zwischen 40 Pa und 150 Pa und einer Vorspannung zwischen 600 V und 800 V aufrechterhalten wird.

12. Herstellungsverfahren nach Anspruch 8, **dadurch gekennzeichnet, dass** in Schritt 4 nach der elektrochemischen Polierbehandlung die chemische Polierbehandlung durchgeführt wird, um 15 bis 25 µm der Dicke der Außenschicht der vorgefertigten Komponente zu entfernen.

13. Herstellungsverfahren nach Anspruch 12, **dadurch gekennzeichnet, dass** in Schritt 4 eine Dreisäuresystem-Polierlösung mit einer Temperatur zwischen 100°C und 180°C bei der chemischen Polierbehandlung angewendet wird und die Dreisäuresystem-Polierlösung die folgenden Hauptbestandteile umfasst: 30% bis 55% H₃PO₄, 20% bis 45% H₂SO₄, 5% bis 15% HNO₃ und 15% bis 30% Wasser.

14. Herstellungsverfahren nach Anspruch 13, **dadurch gekennzeichnet, dass** in Schritt 4 die Zeit für die chemische Polierbehandlung zwischen 3 Sekunden und 8 Sekunden beträgt.

## Revendications

1. Stent vasculaire bio-absorbable, comprenant un composant absorbable produit en soumettant un composant préfabriqué réalisé en une matière brute à base de fer à un traitement de nitruration ionique, la composition de la matière à l'intérieur du composant absorbable changeant avec la profondeur par rapport à la surface, et
le composant absorbable comprenant au moins une première partie et une deuxième partie, la deuxième partie étant entourée par la première partie, et la dureté de la première partie étant supérieure à celle de la deuxième partie,
**caractérisé en ce que** la première partie comprend une couche de dispersion (10) distribuée en continu vers l'intérieur à partir de sa surface, mais dépourvue à sa surface d'une couche (50) résistante à la corrosion formée de nitrures de phase γ' et de phase ε, la couche de dispersion (10) étant une solution solide avec une teneur en azote inférieure à 1% en poids, et des particules avec une teneur en azote supérieure à 1% en poids étant répandues dans la solution solide,
la deuxième partie comprend une couche de solution solide (20) entourée par la couche de dispersion (10), de sorte que la première partie comprend la totalité de la couche de dispersion (10) et la deuxième partie comprend la totalité de la couche de solution solide (20),
la couche (50) résistante à la corrosion étant entièrement enlevée du composant, et
la couche de dispersion (10) et la couche de solution solide (20) étant respectivement agencées d'un des côtés d'une interface formée pour empêcher des fissures générées dans la couche de dispersion (10) de se propager dans la couche de solution solide (20).

2. Stent vasculaire bio-absorbable selon la revendication 1, **caractérisé en ce que** le pourcentage en poids d'azote dans la couche de dispersion (10) est compris entre 1% et 3,7%.

3. Stent vasculaire bio-absorbable selon la revendication 1, **caractérisé en ce que** les particules représentent moins de 63% en poids de la couche de dispersion (10).

4. Stent vasculaire bio-absorbable selon la revendication 3, **caractérisé en ce que** la plupart des particules ont une taille comprise entre 30 nm et 2 µm et la partie présentant la dureté maximale du composant absorbable est agencée près de la surface du composant absorbable et la dureté Vickers maximale n'est pas supérieure à 350HV0,01 ; la dureté de chaque partie de la couche de dispersion (10) étant augmentée de 50HV0,01 par rapport à la dureté Vickers d'une matière brute non nitrurée à base de fer du composant préfabriqué.

5. Stent vasculaire bio-absorbable selon la revendication 4, **caractérisé en ce que** la dureté Vickers de la couche de dispersion (10) est supérieure à 220HV0,01 et inférieure à 320HV0,01 et diminue progressivement avec la profondeur.

6. Stent vasculaire bio-absorbable selon la revendication 1, **caractérisé en ce que** le composant absorbable comprend une pluralité d'entretoises reliées de manière à former une grille tubulaire ; le stent vasculaire est fabriqué en soumettant un tube à base de fer présentant une épaisseur de paroi uniforme à un traitement de gravure et de nitruration ionique et l'épaisseur de la paroi du stent vasculaire est comprise entre 60 µm et 300 µm ; la résistance radiale du stent vasculaire dans des conditions d'une même grille tubulaire et de la même épaisseur de paroi est augmentée de plus de 30% par rapport à la résistance radiale du stent vasculaire non nitruré préfabriqué avec la matière brute à base de fer.

7. Stent vasculaire bio-absorbable selon la revendication 6, **caractérisé en ce que** le stent vasculaire présente une épaisseur de paroi comprise entre 60 µm et 300 µm et une masse comprise entre 5 mg et 100 mg, en particulier une épaisseur de paroi comprise entre 60 µm et 100 µm ou une épaisseur comprise entre 160 µm et 170 µm, et
une dureté Vickers du stent vasculaire étant comprise entre 280HV0,01 et 320HV0,01 ; la résistance radiale du stent vasculaire dans des conditions d'une même grille tubulaire et de la même épaisseur de paroi étant augmentée de plus de 80% par rapport à la résistance radiale du stent vasculaire non nitruré préfabriqué avec la matière brute à base de fer.

8. Procédé de fabrication du stent vasculaire bio-absorbable selon les revendications 1 à 7, comprenant les étapes suivantes :
étape 1. traitement d'un composant préfabriqué en utilisant du fer pur ou un alliage de fer contenant plus de 99 % en poids de fer en tant que matière brute, une épaisseur initiale du composant préfabriqué étant supérieure de 10 µm à une épaisseur correspondante du stent vasculaire ;
étape 2. nettoyage pour éliminer les impuretés sur une surface de l'élément préfabriqué ;
étape 3. traitement du composant préfabriqué de l'étape 2 par application d'un procédé de nitruration ionique, la température du composant préfabriqué étant comprise entre 430°C et 550°Côté, et le temps de nitruration étant compris entre 10 minutes et 200 minutes ;
une couche (50) résistante à la corrosion et riche en azote étant réalisée sur la surface du composant pendant l'étape 3 de nitruration, et
étape 4. un composant préfabriqué nitruré est soumis à un traitement de polissage électrochimique et/ou à un traitement de polissage chimique pour entièrement enlever la couche (50) résistante à la corrosion du composant préfabriqué.

9. Procédé de fabrication selon la revendication 8, **caractérisé en ce qu'**à l'étape 1, la dureté Vickers de surface du composant préfabriqué est comprise entre 165 HV0,01 et 175HV0,01.

10. Procédé de fabrication selon la revendication 8, **caractérisé en ce que** qu'à l'étape 1, une épaisseur du composant préfabriqué est de 20 à 60 µm supérieure à une épaisseur correspondante du stent vasculaire final.

11. Procédé de fabrication selon la revendication 8, **caractérisé en ce que** qu'à l'étape 3, la température du composant préfabriqué est comprise entre 470°C et 520°C, et **en ce qu'**à l'étape 3, un gaz mixte d'azote et d'hydrogène est utilisé dans le procédé de nitruration ionique, et un rapport d'écoulement de l'azote à l'hydrogène est compris entre 1:2 et 1:9, et une décharge de gaz est maintenue à une pression de gaz comprise entre 40 Pa et 150 Pa et à une tension de polarisation comprise entre 600 V et 800 V.

12. Procédé de fabrication selon la revendication 8, **caractérisé en ce qu'**à l'étape 4, après le traitement de polissage électrochimique, le traitement de polissage chimique est réalisé pour enlever 15 à 25 µm de l'épaisseur de la couche extérieure du composant préfabriqué.

13. Procédé de fabrication selon la revendication 12, **caractérisé en ce qu'**à l'étape 4, une solution de polissage à système triacide à une température comprise entre 100°C et 180°C est utilisée dans le traitement de polissage chimique, et la solution de polissage à système triacide comprend les composants principaux suivants : 30% à 55% de H₃PO₄, 20% à 45% de H₂SO₄, 5% à 15% de HNO₃, et 15% à 30% d'eau.

14. Procédé de fabrication selon la revendication 13, **caractérisé en ce qu'**à l'étape 4, le temps du traitement de polissage chimique est compris entre 3 secondes et 8 secondes.
